(19) Europäisches Patentamt
European Patent Office
Office européen des brevets

(11) Veröffentlichungsnummer: **0 017 850**
**B1**

# (12) EUROPÄISCHE PATENTSCHRIFT

(45) Veröffentlichungstag der Patentschrift: **08.12.82**

(21) Anmeldenummer: **80101749.2**

(22) Anmeldetag: **02.04.80**

(51) Int. Cl.³: **C 07 C 103/48**, C 07 C 143/68, C 07 C 149/23, C 07 D 231/12, C 07 D 249/08, C 07 D 307/68, C 07 D 309/12, A 01 N 37/46, A 01 N 41/04, A 01 N 43/08 // A01N43/16, A01N43/56, A01N43/64

(54) **N,N-Disubstituierte Ethylglycinester, ihre Herstellung und Verwendung, sie enthaltende Fungizide und deren Herstellung.**

(30) Priorität: **12.04.79 DE 2915026**

(43) Veröffentlichungstag der Anmeldung: **29.10.80 Patentblatt 80/22**

(45) Bekanntmachung des Hinweises auf die Patenterteilung: **08.12.82 Patentblatt 82/49**

(84) Benannte Vertragsstaaten: **AT BE CH DE FR GB IT LI NL SE**

(56) Entgegenhaltungen:
**AT-B-347 182**
**DE-A-2 702 102**
**DE-B-2 513 788**
**FR-A-2 326 416**
**GB-A-1 520 776**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Lunkenheimer, Winfried, Dr., Bismarckstrasse 29, D-5600 Wuppertal 1 (DE)**
Erfinder: **Brandes, Wilhelm, Dr., Eichendorffstrasse 3, D-5653 Leichlingen 1 (DE)**

**EP 0 017 850 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

N,N-disubstituierte Ethylglycinester, ihre Herstellung und Verwendung sie enthaltende Fungizide und deren Herstellung

Die vorliegende Erfindung betrifft neue N,N-disubstituierte Ethylglycinester, mehrere Verfahren zu ihrer Herstellung sowie ihre Verwendung als Fungizide.

Es ist bereits bekanntgeworden, daß Halogenacetanilide, wie beispielsweise N-Chloracetyl-N-(2,6-dialkylphenyl)-alanin- bzw. glycin-alkylester mit gutem Erfolg zur Bekämpfung von pilzlichen Pflanzenkrankheiten eingesetzt werden können (vergleiche DE-OS 2 350 944 bzw. US-Patentschrift 3 780 090). Weiterhin sind aus GB-PS 1 520 776, FR-A-2 326 416, DE-AS 2 513 788, DE-OS 2 702 102 und AT-PS 347 182 N-substituierte Acetanilide und deren fungizide Wirksamkeit bekannt. Deren Wirkung ist jedoch, insbesondere bei niedrigen Aufwandmengen und -konzentrationen, insbesondere auch bei der Bekämpfung von Phytophtora-Arten, nicht immer ganz befriedigend.

Es wurden neue N,N-disubstituierte Ethylglycinester der allgemeinen Formel

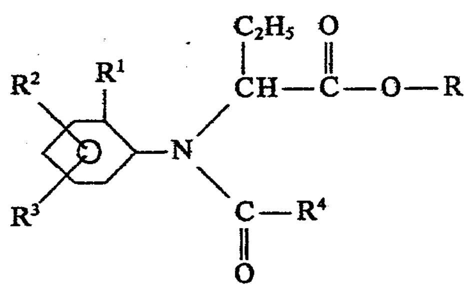

(I)

in welcher

R für Alkyl, Hydroxyalkyl, Cyanalkyl, Alkenyl, Alkinyl, Halogenalkyl, Cycloalkyl, Cycloalkylalkyl, Alkoxyalkyl, Alkoxyalkoxyalkyl, Acyloxyalkyl, Alkylthioalkyl, Alkylsulfinylalkyl, Alkylsulfonylalkyl, Dialkylaminoalkyl oder gegebenenfalls substituiertes Aralkyl steht,

$R^1$ für Wasserstoff, Alkyl, Alkoxy oder Halogen steht,

$R^2$ für Wasserstoff, Alkyl, Alkoxy oder Halogen steht,

$R^3$ für Wasserstoff oder Alkyl steht,

$R^4$ für Furyl, Tetrahydrofuryl, Thienyl, Tetrahydrothienyl; gegebenenfalls durch Alkyl substituiertes Isoxazolyl; Hydroxyalkyl; gegebenenfalls durch Cyano oder Thiocyano substituiertes Alkyl, Alkenyl und Alkinyl; Cycloalkyl; Dihalogenalkyl; sowie die Gruppierungen

$-CH_2-Az$ $-CH_2-OR^5$ $-CH_2-SR^5$ $-OR^5$ $-SR^5$

$-CH_2-OSO_2R^5$ $-CH_2OCOR^5$ und $-CH_2-O-$ 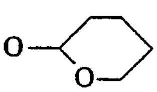

steht, wobei

$R^5$ für gegebenenfalls substituiertes Alkyl, Alkenyl, Alkinyl und Alkoxyalkyl steht, und

Az für Pyrazol-1-yl, 1,2,4-Triazol-1-yl und Imidazol-1-yl steht,

gefunden.

Weiterhin wurde gefunden, daß man die N,N-disubstituierten Ethylglycinester der Formel (I) erhält, wenn man

a) N-Phenyl-ethylglycinester der Formel

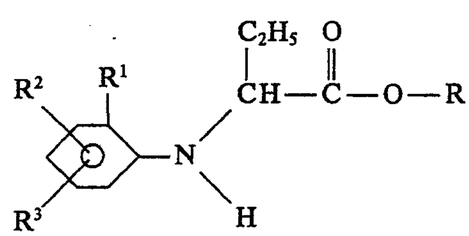

(II)

in welcher

R, $R^1$, $R^2$ und $R^3$ die oben angegebene Bedeutung haben,

mit Säurechloriden oder -bromiden bzw. -anhydriden der Formeln

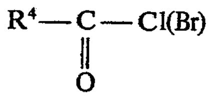

(IIIa)

bzw.

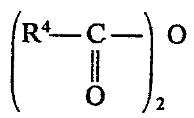

(IIIb)

in welchen

$R^4$ die oben angegebene Bedeutung hat,

in Gegenwart eines Verdünnungsmittels und gegebenenfalls in Gegenwart eines Säurebindemittels umsetzt, oder

b) N-Acyl-N-phenyl-ethylglycine der Formel

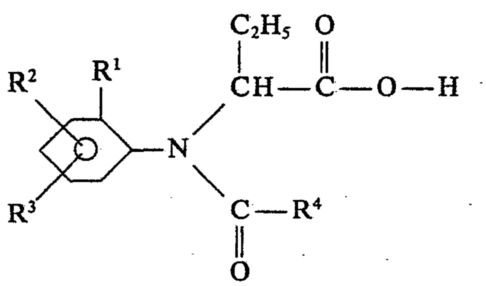

(IV)

in welcher

$R^1$, $R^2$, $R^3$ und $R^4$ die oben angegebene Bedeutung haben,

mit Alkoholen der Formel

$$R-O-H \qquad (V)$$

in welcher

R die oben angegebene Bedeutung hat,

in Gegenwart eines Kondensationsmittels und gegebenenfalls in Gegenwart eines Verdünnungsmittels umsetzt, oder

c) N-Acyl-N-phenyl-ethylglycinchloride der Formel

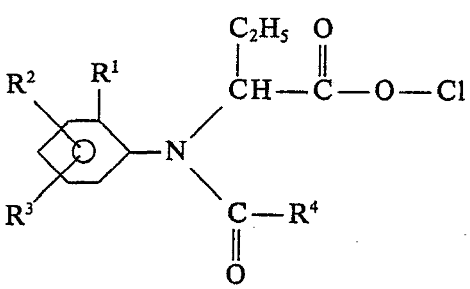

(VI)

in welcher

$R^1$, $R^2$, $R^3$ und $R^4$ die oben angegebene Bedeutung haben,

mit Alkoholen der Formel (V) in Gegenwart eines Verdünnungsmittels und gegebenenfalls in Gegenwart einer Base umsetzt, oder zur Darstellung einzelner Verbindungen der Formel (I)

d) N-Halogenacetyl-N-phenyl-ethylglycinester der Formel

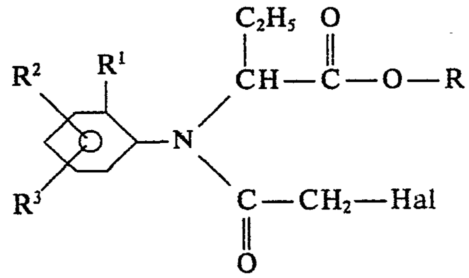

(VII)

in welcher

R, $R^1$, $R^2$ und $R^3$ die oben angegebene Bedeutung haben und
Hal für Chlor, Brom oder Jod steht,

mit Verbindungen der Formel

B—X (VIII)

in welcher

X für Az, Cyano, Thiocyano, und die Gruppierung $-OR^5$ oder $-SR^5$ oder Alkylcarbonyloxy mit 1 bis 4 Kohlenstoffatomen im Alkylteil steht und
B für Wasserstoff oder ein Alkalimetall steht,

in Gegenwart eines Verdünnungsmittels und gegebenenfalls in Gegenwart eines Säurebinders umsetzt, oder

e) erfindungsgemäße N-Hydroxyacetyl-N-phenyl-ethylglycinester der Formel

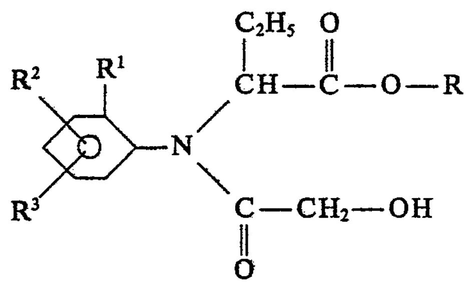

(IX)

in welcher

R, $R^1$ $R^2$ und $R^3$ die oben angegebene Bedeutung haben,

(1) gegebenenfalls nach Altivierung mittels Alkalimetall mit Halogeniden der Formel

Hal—$R^6$ (X)

in welcher

Hal die oben angegebene Bedeutung hat, und
$R^6$ für den Rest $R^5$ sowie die Gruppen $-SO_2R^5$ und $-COR^5$ steht, wobei
$R^5$ die oben angegebene Bedeutung hat,

in Gegenwart eines Verdünnungsmittels und gegebenenfalls in Gegenwart eines Säurebinders umsetzt, oder

(2) mit Dihydropyran der Formel

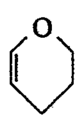

(XI)

in Gegenwart eines Verdünnungsmittels und gegebenenfalls in Gegenwart eines Katalysators umsetzt.

Außerdem wurde gefunden, daß die neuen N,N-disubstituierten Ethylglycinester der Formel (I) starke fungizide Eigenschaften aufweisen.

Überraschenderweise zeigen die erfindungsgemäßen N,N-disubstituierten Ethylglycinester eine erheblich höhere Wirkung, insbesondere gegen Phytophthora, als die aus dem Stand der Technik bekannten N-Chloracetyl-N-(2,6-dialkylphenyl)-alanin- bzw. -glycinalkylester, welche chemisch und wirkungsmäßig naheliegende Verbindungen sind. Die erfindungsgemäßen Wirkstoffe stellen eine Bereicherung der Technik dar.

Die erfindungsgemäßen N,N-disubstituierten Ethylglycinester sind durch die Formel (I) allgemein definiert. In dieser Formel steht R vorzugsweise für geradkettiges oder verzweigtes Alkyl mit 1 bis 6 Kohlenstoffatomen; für Hydroxy- und Cyanalkyl mit jeweils 1 bis 4 Kohlenstoffatomen im Alkylteil; für Alkenyl und Alkinyl mit 2 bis 4 Kohlenstoffatomen und für Halogenalkyl mit 1 bis 4 Kohlenstoff- und bis zu 5 Halogenatomen, insbesondere mit bis zu 2 Kohlenstoff- und bis zu 3 gleichen oder verschiedenen Halogenatomen, wobei als Halogene insbesondere Fluor und Chlor stehen. R steht weiterhin vorzugsweise für Cycloalkyl und Cycloalkylalkyl mit 3 bis 7 Kohlenstoffatomen in jedem Cycloalkylteil und 1 bis 4 Kohlenstoffatomen im Alkylteil; für Alkoxyalkyl, Alkoxyalkoxyalkyl, Alkylthioalkyl, Alkylsulfinylalkyl, Alkylsulfonylalkyl und Dialkylaminoalkyl mit 1 bis 4 Kohlenstoffatomen in jedem Alkylteil, sowie vorzugsweise für Acyloxyalkyl mit 1 bis 4 Kohlenstoffatomen im Alkylteil, wobei im Acylteil mit der allgemeinen Formel $R^7—CO—$ der Rest $R^7$ vorzugsweise bedeutet: Alkyl mit 1 bis 4 Kohlenstoffatomen; Halogenalkyl mit 1 bis 4 Kohlenstoff- und bis zu 5 Halogenatomen, insbesondere mit bis zu 2 Kohlenstoff- und bis zu 3 gleichen oder verschiedenen Halogenatomen, wobei als Halogene insbesondere Fluor und Chlor stehen; und gegebenenfalls im Arylteil substituiertes Aryl oder Arylalkyl mit 6 bis 10 Kohlenstoffatomen im Arylteil und 1 bis 4 Kohlenstoffatomen im Alkylteil, wie insbesondere Phenyl oder Benzyl, wobei als Substituenten vorzugsweise infrage kommen: Halogen, insbesondere Fluor, Chlor und Brom; Cyano, Nitro, Alkyl mit 1 bis 2 Kohlenstoffatomen sowie Halogenalkyl mit bis zu zwei Kohlenstoff- und bis zu 3 gleichen oder verschiedenen Halogenatomen, wobei als Halogene insbesondere Fluor oder Chlor stehen, beispielhaft sei Trifluormethyl genannt. R steht außerdem vorzugsweise für gegebenenfalls im Arylteil substituiertes Aralkyl mit 6 bis 10 Kohlenstoffatomen im Arylteil, insbesondere Phenyl, und 1 bis 4 Kohlenstoffatomen im Alkylteil, wobei als Substituenten vorzugsweise infrage kommen: Halogene, insbesondere Fluor, Chlor und Brom; Cyano, Nitro, Alkyl mit 1 oder 2 Kohlenstoffatomen sowie Halogenalkyl mit bis zu 2 Kohlenstoff- und bis zu 3 gleichen oder verschiedenen Halogenatomen, wobei als Halogene insbesondere Fluor und Chlor stehen, beispielhaft sei Trifluormethyl genannt. $R^1$ und $R^2$ stehen vorzugsweise für Wasserstoff, geradkettiges oder verzweigtes Alkyl oder Alkoxy mit 1 bis 4 Kohlenstoffatomen, sowie für Halogen, insbesondere Fluor, Chlor oder Brom. $R^3$ steht vorzugsweise für Wasserstoff und geradkettiges oder verzweigtes Alkyl mit 1 bis 4 Kohlenstoffatomen. $R^4$ steht vorzugsweise für Furyl, Tetrahydrofuryl, Thienyl, Tetrahydrothienyl, für gegebenenfalls durch Methyl oder Ethyl substituiertes Isoxazolyl, für Hydroxylalkyl mit 1 oder 2 Kohlenstoffatomen, für gegebenenfalls durch Cyano oder Thiocyano substituiertes Alkyl mit 1 bis 4 und Alkenyl sowie Alkinyl mit jeweils 2 bis 4 Kohlenstoffatomen, sowie für Cycloalkyl mit 3 bis 7 Kohlenstoffatomen; ferner vorzugsweise für Dihalogenalkyl mit 1 bis 2 Kohlenstoffatomen, wobei als Halogenatome vorzugsweise Fluor und Chlor genannt seien, sowie für die Gruppierungen

$$—CH_2—Az \qquad —CH_2—OR^5 \qquad —CH_2—SR^5 \qquad —OR^5 \qquad —SR^5$$

$$—CH_2—OSO_2R^5 \qquad —CH_2OCOR^5$$ und $—CH_2—O—$ 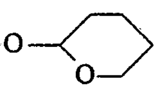

Az steht dabei für Pyrazol-1-yl, 1,2,4-Triazol-1-yl und Imidazol-1-yl. $R^5$ steht vorzugsweise für gegebenenfalls durch Halogen, insbesondere Fluor, Chlor und Brom, Cyano und Thiocyano substituiertes Alkyl mit 1 bis 4 Alkenyl sowie Alkinyl mit jeweils 2 bis 4 Kohlenstoffatomen und für Alkoxyalkyl mit 1 bis 4 Kolenstoffatomen in jedem Alkylteil.

Ganz besonders bevorzugt sind diejenigen Verbindungen der Formel (I) in denen R für Methyl, Ethyl, n-Propyl, Isopropyl, n-Butyl, Isobutyl, sek.-Butyl, tert.-Butyl, Hydroxyethyl, Cyanmethyl, Allyl, Propargyl, Chlorethyl, Cyclopropyl, Cyclopropylmethyl, Cyclohexyl, Cyclohexylmethyl, Methoxyethyl, Ethoxyethyl, Methylthioethyl, Ethylthioethyl, Methoxyethoxyethyl, Chloracetoxyethyl, Methylsulfinylethyl, Ethylsulfinylethyl, Methylsulfonylethyl, Ethylsulfonylethyl, gegebenenfalls durch Chlor, Methyl und/oder Ethyl substituiertes Benzyl sowie Dimethylaminoethyl oder Diethylaminoethyl steht; $R^1$, $R^2$ und $R^3$ gleich oder verschieden sind, und für Wasserstoff, Methyl, Ethyl, n-Propyl, Isopropyl, n-Butyl, Isobutyl, sek.-Butyl oder tert.-Butyl stehen; $R^1$ und $R^2$ außerdem auch für Methoxy, Ethoxy, Isopropoxy, Fluor, Chlor oder Brom stehen; und $R^4$ für 2-Furyl, 2-Thienyl, 2-Tetrahydrofuryl, 5-Methylisoxazol-3-yl, Methoxymethyl, Ethoxymethyl, Allyloxymethyl, Propargyloxymethyl, Ethoxymethoxymethyl, Methylmercaptomethyl, Methoxy, Ethoxy, Methylmercapto, Cyclohexyl, Dichlormethyl, Hydroxymethyl, Methansulfonyloxymethyl, Acetoxymethyl, Propionyloxymethyl, Dichlormethyl, Pyrazol-1-yl-methyl, Imidazol-1-yl-methyl, 1,2,4-Triazol-1-yl-methyl und Tetrahydropyran-2-yloxymethyl steht.

Im einzelnen seien außer den bei den Herstellungsbeispielen genannten Verbindungen die folgenden Verbindungen der allgemeinen Formel (I) genannt:

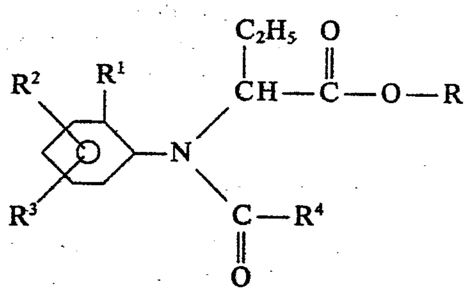

(I)

| $R^1$ | $R^2$ | $R^3$ | $R^4$ | R |
|---|---|---|---|---|
| H | H | H | 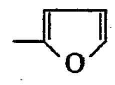 | $-CH_3$ |
| H | H | H | 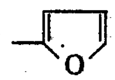 | $-C_2H_5$ |
| H | H | H | 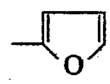 | $-CH_2CH_2Cl$ |
| H | H | H | 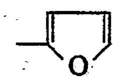 | $-CH_2CH_2OCH_3$ |
| H | H | H | 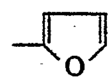 | $-CH_2CH_2OC_2H_4OCH_3$ |
| H | H | H | 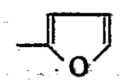 | $-CH_2CH_2OCOCH_2Cl$ |
| H | H | H | 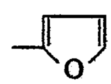 | $-CH_2-CH=CH_2$ |
| H | H | H | 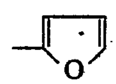 | $-CH_2-C\equiv CH$ |
| H | H | H | 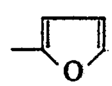 | 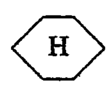 |
| H | H | H | 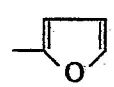 | $-CH_2-$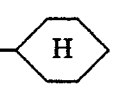 |
| H | H | H | 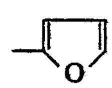 | $-CH_2CH_2SCH_3$ |
| H | H | H | 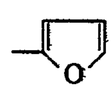 | $-CH_2CH_2SOCH_3$ |
| H | H | H | 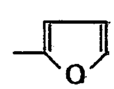 | $-CH_2CH_2SO_2CH_3$ |

Fortsetzung

| $R^1$ | $R^2$ | $R^3$ | $R^4$ | R |
|---|---|---|---|---|
| H | H | H | 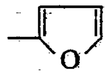 | $—CH_2CH_2—N(CH_3)_2$ |
| H | H | H | 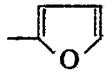 | $—CH_2—$ 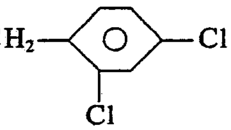 |
| H | H | H | 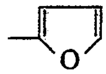 | $—CH_2CN$ |
| $CH_3$ | 6-$CH_3$ | H | 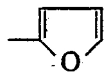 | $—C_2H_5$ |
| $CH_3$ | 6-$CH_3$ | H | 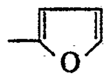 | $—CH_2CH_2Cl$ |
| $CH_3$ | 6-$CH_3$ | H | 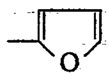 | $—CH_2CH_2OCH_3$ |
| $CH_3$ | 6-$CH_3$ | H | 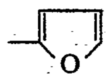 | $—CH_2CH_2OC_2H_4OCH_3$ |
| $CH_3$ | 6-$CH_3$ | H | 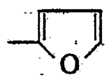 | $—CH_2CH_2OCOCH_2Cl$ |
| $CH_3$ | 6-$CH_3$ | H | 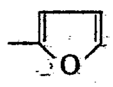 | $—CH_2—CH{=}CH_2$ |
| $CH_3$ | 6-$CH_3$ | H | 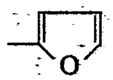 | $—CH_2—C{\equiv}CH$ |
| $CH_3$ | 6-$CH_3$ | H | 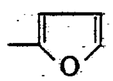 | 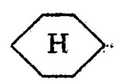 |
| $CH_3$ | 6-$CH_3$ | H | 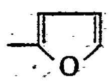 | $—CH_2—$ 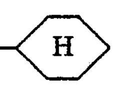 |
| $CH_3$ | 6-$CH_3$ | H | 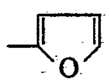 | $—CH_2CH_2SCH_3$ |
| $CH_3$ | 6-$CH_3$ | H | 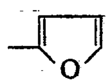 | $—CH_2CH_2SOCH_3$ |
| $CH_3$ | 6-$CH_3$ | H | 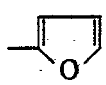 | $—CH_2CH_2SO_2CH_3$ |
| $CH_3$ | 6-$CH_3$ | H | 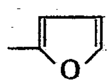 | $—CH_2CH_2—N(CH_3)_2$ |

Fortsetzung

| $R^1$ | $R^2$ | $R^3$ | $R^4$ | R |
|---|---|---|---|---|
| $CH_3$ | 6-$CH_3$ | H | 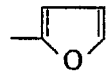 | 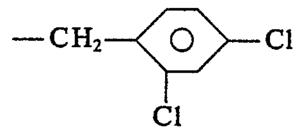 |
| $CH_3$ | 6-$CH_3$ | H | 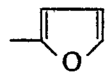 | $-CH_2CN$ |
| $CH_3$ | 6-$C_2H_5$ | H | 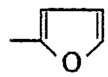 | $-CH_3$ |
| $CH_3$ | 6-$C_2H_5$ | H | 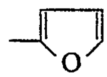 | $-C_2H_5$ |
| $CH_3$ | 6-$C_2H_5$ | H | 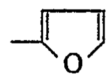 | $-CH_2CH_2Cl$ |
| $CH_3$ | 6-$C_2H_5$ | H | 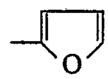 | $-CH_2CH_2OCH_3$ |
| $CH_3$ | 6-$C_2H_5$ | H | 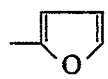 | $-CH_2CH_2OC_2H_4OCH_3$ |
| $CH_3$ | 6-$C_2H_5$ | H | 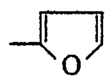 | $-CH_2CH_2OCOCH_2Cl$ |
| $CH_3$ | 6-$C_2H_5$ | H | 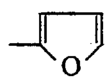 | $-CH_2-CH=CH_2$ |
| $CH_3$ | 6-$C_2H_5$ | H | 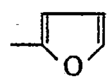 | $-CH_2-C\equiv CH$ |
| $CH_3$ | 6-$C_2H_5$ | H | 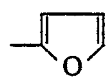 | 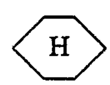 |
| $CH_3$ | 6-$C_2H_5$ | H | 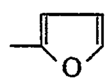 | 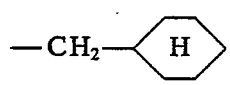 |
| $CH_3$ | 6-$C_2H_5$ | H | 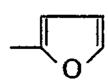 | $-CH_2CH_2SCH_3$ |
| $CH_3$ | 6-$C_2H_5$ | H | 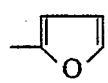 | $-CH_2CH_2SOCH_3$ |
| $CH_3$ | 6-$C_2H_5$ | H | 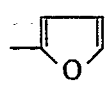 | $-CH_2CH_2SO_2CH_3$ |
| $CH_3$ | 6-$C_2H_5$ | H | 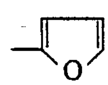 | $-CH_2CH_2-N(CH_3)_2$ |

Fortsetzung

| $R^1$ | $R^2$ | $R^3$ | $R^4$ | R |
|---|---|---|---|---|
| $CH_3$ | 6-$C_2H_5$ | H | 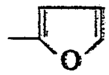 | 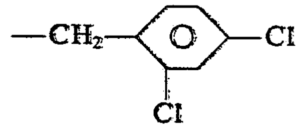 |
| $CH_3$ | 6-$C_2H_5$ | H | 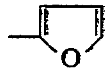 | $-CH_2CN$ |
| H | H | H | $-CH_2-O-CH_3$ | $-CH_3$ |
| H | H | H | $-CH_2-O-CH_3$ | $-C_2H_5$ |
| H | H | H | $-CH_2-O-CH_3$ | $-CH_2CH_2Cl$ |
| H | H | H | $-CH_2-O-CH_3$ | $-CH_2CH_2OCH_3$ |
| H | H | H | $-CH_2-O-CH_3$ | $-CH_2CH_2OC_2H_4OCH_3$ |
| H | H | H | $-CH_2-O-CH_3$ | $-CH_2CH_2OCOCH_2Cl$ |
| H | H | H | $-CH_2-O-CH_3$ | $-CH_2-CH=CH_2$ |
| H | H | H | $-CH_2-O-CH_3$ | $-CH_2-C\equiv CH$ |
| H | H | H | $-CH_2-O-CH_3$ | 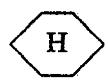 |
| H | H | H | $-CH_2-O-CH_3$ | 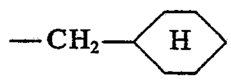 |
| H | H | H | $-CH_2-O-CH_3$ | $-CH_2CH_2SCH_3$ |
| H | H | H | $-CH_2-O-CH_3$ | $-CH_2CH_2SOCH_3$ |
| H | H | H | $-CH_2-O-CH_3$ | $-CH_2CH_2SO_2CH_3$ |
| H | H | H | $-CH_2-O-CH_3$ | $-CH_2CH_2-N(CH_3)_2$ |
| H | H | H | $-CH_2-O-CH_3$ | 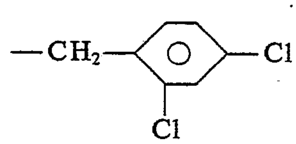 |
| H | H | H | $-CH_2-O-CH_3$ | $-CH_2CN$ |
| $CH_3$ | 6-$CH_3$ | H | $-CH_2-O-CH_3$ | $-C_2H_5$ |
| $CH_3$ | 6-$CH_3$ | H | $-CH_2-O-CH_3$ | $-CH_2CH_2Cl$ |
| $CH_3$ | 6-$CH_3$ | H | $-CH_2-O-CH_3$ | $-CH_2CH_2OCH_3$ |
| $CH_3$ | 6-$CH_3$ | H | $-CH_2-O-CH_3$ | $-CH_2CH_2OC_2H_4OCH_3$ |
| $CH_3$ | 6-$CH_3$ | H | $-CH_2-O-CH_3$ | $-CH_2CH_2OCOCH_2Cl$ |
| $CH_3$ | 6-$CH_3$ | H | $-CH_2-O-CH_3$ | $-CH_2-CH=CH_2$ |

Fortsetzung

| $R^1$ | $R^2$ | $R^3$ | $R^4$ | R |
|---|---|---|---|---|
| $CH_3$ | 6-$CH_3$ | H | $-CH_2-O-CH_3$ | $-CH_2-C{\equiv}CH$ |
| $CH_3$ | 6-$CH_3$ | H | $-CH_2-O-CH_3$ | 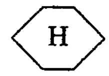 |
| $CH_3$ | 6-$CH_3$ | H | $-CH_2-O-CH_3$ | $-CH_2-$ 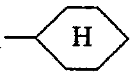 |
| $CH_3$ | 6-$CH_3$ | H | $-CH_2-O-CH_3$ | $-CH_2CH_2SCH_3$ |
| $CH_3$ | 6-$CH_3$ | H | $-CH_2-O-CH_3$ | $-CH_2CH_2SOCH_3$ |
| $CH_3$ | 6-$CH_3$ | H | $-CH_2-O-CH_3$ | $-CH_2CH_2SO_2CH_3$ |
| $CH_3$ | 6-$CH_3$ | H | $-CH_2-O-CH_3$ | $-CH_2CH_2-N(CH_3)_2$ |
| $CH_3$ | 6-$CH_3$ | H | $-CH_2-O-CH_3$ | $-CH_2-$ 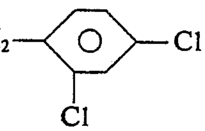 |
| $CH_3$ | 6-$CH_3$ | H | $-CH_2-O-CH_3$ | $-CH_2CN$ |
| $CH_3$ | 6-$C_2H_5$ | H | $-CH_2-O-CH_3$ | $-CH_3$ |
| $CH_3$ | 6-$C_2H_5$ | H | $-CH_2-O-CH_3$ | $-C_2H_5$ |
| $CH_3$ | 6-$C_2H_5$ | H | $-CH_2-O-CH_3$ | $-CH_2CH_2Cl$ |
| $CH_3$ | 6-$C_2H_5$ | H | $-CH_2-O-CH_3$ | $-CH_2CH_2OCH_3$ |
| $CH_3$ | 6-$C_2H_5$ | H | $-CH_2-O-CH_3$ | $-CH_2CH_2OC_2H_4OCH_3$ |
| $CH_3$ | 6-$C_2H_5$ | H | $-CH_2-O-CH_3$ | $-CH_2CH_2OCOCH_2Cl$ |
| $CH_3$ | 6-$C_2H_5$ | H | $-CH_2-O-CH_3$ | $-CH_2-CH{=}CH_2$ |
| $CH_3$ | 6-$C_2H_5$ | H | $-CH_2-O-CH_3$ | $-CH_2-C{\equiv}CH$ |
| $CH_3$ | 6-$C_2H_5$ | H | $-CH_2-O-CH_3$ | 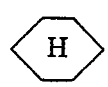 |
| $CH_3$ | 6-$C_2H_5$ | H | $-CH_2-O-CH_3$ | $-CH_2-$ 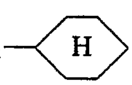 |
| $CH_3$ | 6-$C_2H_5$ | H | $-CH_2-O-CH_3$ | $-CH_2CH_2SCH_3$ |
| $CH_3$ | 6-$C_2H_5$ | H | $-CH_2-O-CH_3$ | $-CH_2CH_2SOCH_3$ |
| $CH_3$ | 6-$C_2H_5$ | H | $-CH_2-O-CH_3$ | $-CH_2CH_2SO_2CH_3$ |
| $CH_3$ | 6-$C_2H_5$ | H | $-CH_2-O-CH_3$ | $-CH_2CH_2-N(CH_3)_2$ |
| $CH_3$ | 6-$C_2H_5$ | H | $-CH_2-O-CH_3$ | $-CH_2-$ 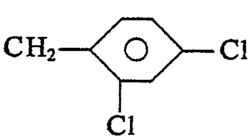 |

Fortsetzung

| $R^1$ | $R^2$ | $R^3$ | $R^4$ | R |
|---|---|---|---|---|
| $CH_3$ | 6-$C_2H_5$ | H | $-CH_2-O-CH_3$ | $-CH_2CN$ |
| H | H | H | $-CH_2-$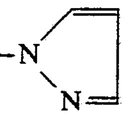 | $-CH_3$ |
| H | H | H | $-CH_2-$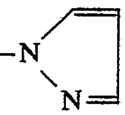 | $-C_2H_5$ |
| H | H | H | $-CH_2-$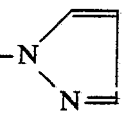 | $-CH_2CH_2Cl$ |
| H | H | H | $-CH_2-$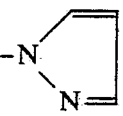 | $-CH_2CH_2OCH_3$ |
| H | H | H | $-CH_2-$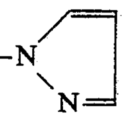 | $-CH_2CH_2OC_2H_4OCH_3$ |
| H | H | H | $-CH_2-$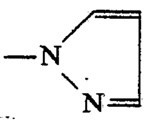 | $-CH_2CH_2OCOCH_2Cl$ |
| H | H | H | $-CH_2-$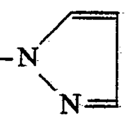 | $-CH_2-CH=CH_2$ |
| H | H | H | $-CH_2-$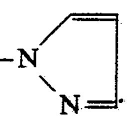 | $-CH_2-C\equiv CH$ |
| H | H | H | $-CH_2-$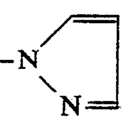 | 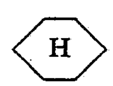 |
| H | H | H | $-CH_2-$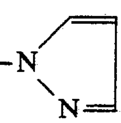 | $-CH_2-$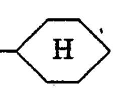 |
| H | H | H | $-CH_2-$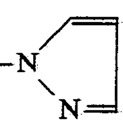 | $-CH_2CH_2SCH_3$ |
| H | H | H | $-CH_2-$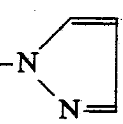 | $-CH_2CH_2SOCH_3$ |

Fortsetzung

| $R^1$ | $R^2$ | $R^3$ | $R^4$ | R |
|---|---|---|---|---|
| H | H | H | $-CH_2-$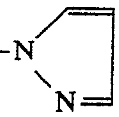 | $-CH_2CH_2SO_2CH_3$ |
| H | H | H | $-CH_2-$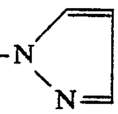 | $-CH_2CH_2-N(CH_3)_2$ |
| H | H | H | $-CH_2-$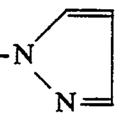 | $-CH_2-$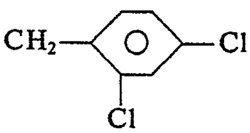 |
| H | H | H | $-CH_2-$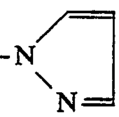 | $-CH_2CN$ |
| $CH_3$ | 6-$CH_3$ | H | $-CH_2-$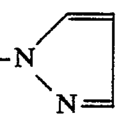 | $-C_2H_5$ |
| $CH_3$ | 6-$CH_3$ | H | $-CH_2-$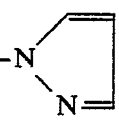 | $-CH_2CH_2Cl$ |
| $CH_3$ | 6-$CH_3$ | H | $-CH_2-$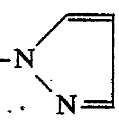 | $-CH_2CH_2OCH_3$ |
| $CH_3$ | 6-$CH_3$ | H | $-CH_2-$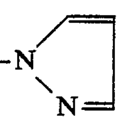 | $-CH_2CH_2OC_2H_4OCH_3$ |
| $CH_3$ | 6-$CH_3$ | H | $-CH_2-$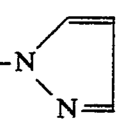 | $-CH_2CH_2OCOCH_2Cl$ |
| $CH_3$ | 6-$CH_3$ | H | $-CH_2-$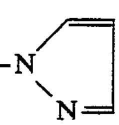 | $-CH_2-CH=CH_2$ |
| $CH_3$ | 6-$CH_3$ | H | $-CH_2-$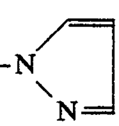 | $-CH_2-C\equiv CH$ |
| $CH_3$ | 6-$CH_3$ | H | $-CH_2-$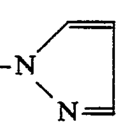 | 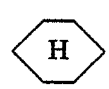 |

Fortsetzung

| $R^1$ | $R^2$ | $R^3$ | $R^4$ | R |
|---|---|---|---|---|
| $CH_3$ | 6-$CH_3$ | H | 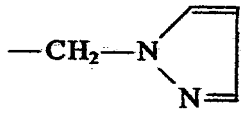 | 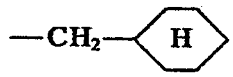 |
| $CH_3$ | 6-$CH_3$ | H | 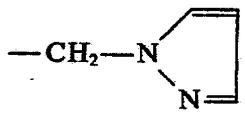 | $-CH_2CH_2SCH_3$ |
| $CH_3$ | 6-$CH_3$ | H | 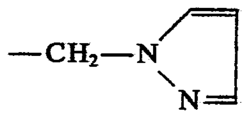 | $-CH_2CH_2SOCH_3$ |
| $CH_3$ | 6-$CH_3$ | H | 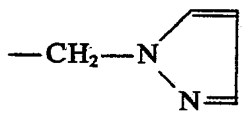 | $-CH_2CH_2SO_2CH_3$ |
| $CH_3$ | 6-$CH_3$ | H | 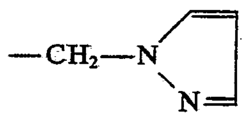 | $-CH_2CH_2-N(CH_3)_2$ |
| $CH_3$ | 6-$CH_3$ | H | 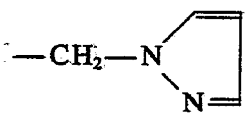 | 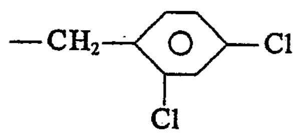 |
| $CH_3$ | 6-$CH_3$ | H | 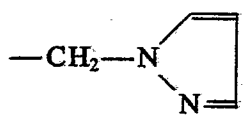 | $-CH_2CN$ |
| $CH_3$ | 6-$C_2H_5$ | H | 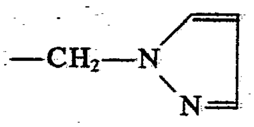 | $-CH_3$ |
| $CH_3$ | 6-$C_2H_5$ | H | 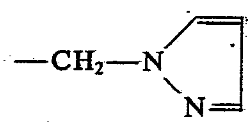 | $-C_2H_5$ |
| $CH_3$ | 6-$C_2H_5$ | H | 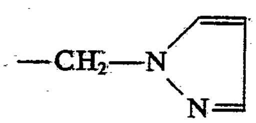 | $-CH_2CH_2Cl$ |
| $CH_3$ | 6-$C_2H_5$ | H | 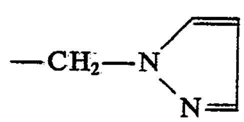 | $-CH_2CH_2OCH_3$ |
| $CH_3$ | 6-$C_2H_5$ | H | 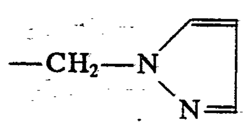 | $-CH_2CH_2OC_2H_4OCH_3$ |

Fortsetzung

| $R^1$ | $R^2$ | $R^3$ | $R^4$ | R |
|---|---|---|---|---|
| $CH_3$ | 6-$C_2H_5$ | H | —$CH_2$— 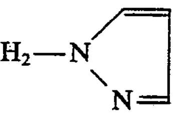 | —$CH_2CH_2OCOCH_2Cl$ |
| $CH_3$ | 6-$C_2H_5$ | H | —$CH_2$— 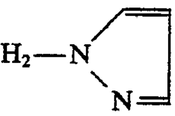 | —$CH_2$—$CH{=}CH_2$ |
| $CH_3$ | 6-$C_2H_5$ | H | —$CH_2$— 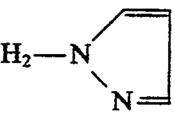 | —$CH_2$—$C{\equiv}CH$ |
| $CH_3$ | 6-$C_2H_5$ | H | —$CH_2$— 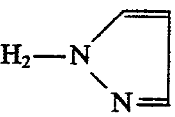 | 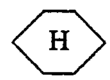 |
| $CH_3$ | 6-$C_2H_5$ | H | —$CH_2$— 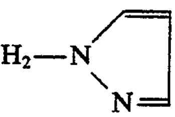 | —$CH_2$— 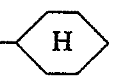 |
| $CH_3$ | 6-$C_2H_5$ | H | —$CH_2$— 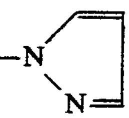 | —$CH_2CH_2SCH_3$ |
| $CH_3$ | 6-$C_2H_5$ | H | —$CH_2$— 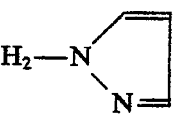 | —$CH_2CH_2SOCH_3$ |
| $CH_3$ | 6-$C_2H_5$ | H | —$CH_2$— 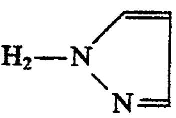 | —$CH_2CH_2SO_2CH_3$ |
| $CH_3$ | 6-$C_2H_5$ | H | —$CH_2$— 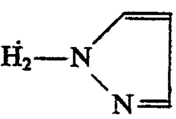 | —$CH_2CH_2$—$N(CH_3)_2$ |
| $CH_3$ | 6-$C_2H_5$ | H | —$CH_2$— 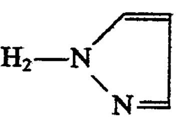 | —$CH_2$— 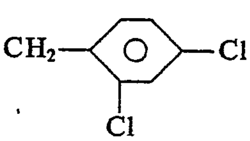 |
| $CH_3$ | 6-$C_2H_5$ | H | —$CH_2$— 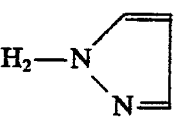 | —$CH_2CN$ |
| H | H | H | —$CHCl_2$ | —$CH_3$ |
| H | H | H | —$CHCl_2$ | —$C_2H_5$ |
| H | H | H | —$CHCl_2$ | —$CH_2CH_2OCH_3$ |

Fortsetzung

| $R^1$ | $R^2$ | $R^3$ | $R^4$ | R |
|---|---|---|---|---|
| H | H | H | $-CHCl_2$ | $-CH_2-CH=CH_2$ |
| H | H | H | $-CHCl_2$ | $-CH_2-CH\equiv CH$ |
| H | H | H | $-CHCl_2$ | $-CH_2CH_2OC_2H_5$ |
| H | H | H | $-CHCl_2$ | $-C_2H_4OC_2H_4OCH_3$ |
| H | H | H | $-CHCl_2$ | $-CH_2CN$ |
| $CH_3$ | 6-$CH_3$ | H | $-CHCl_2$ | $-C_2H_5$ |
| $CH_3$ | 6-$CH_3$ | H | $-CHCl_2$ | $-CH_2CH_2OCH_3$ |
| $CH_3$ | 6-$CH_3$ | H | $-CHCl_2$ | $-CH_2-CH=CH_2$ |
| $CH_3$ | 6-$CH_3$ | H | $-CHCl_2$ | $-CH_2-C\equiv CH$ |
| $CH_3$ | 6-$CH_3$ | H | $-CHCl_2$ | $-CH_2CH_2OC_2H_5$ |
| $CH_3$ | 6-$CH_3$ | H | $-CHCl_2$ | $-C_2H_4OC_2H_4OCH_3$ |
| $CH_3$ | 6-$CH_3$ | H | $-CHCl_2$ | $-CH_2CN$ |
| $CH_3$ | 6-$C_2H_5$ | H | $-CHCl_2$ | $-CH_3$ |
| $CH_3$ | 6-$C_2H_5$ | H | $-CHCl_2$ | $-C_2H_5$ |
| $CH_3$ | 6-$C_2H_5$ | H | $-CHCl_2$ | $-CH_2CH_2OCH_3$ |
| $CH_3$ | 6-$C_2H_5$ | H | $-CHCl_2$ | $-CH_2-CH=CH_2$ |
| $CH_3$ | 6-$C_2H_5$ | H | $-CHCl_2$ | $-CH_2-C\equiv CH$ |
| $CH_3$ | 6-$C_2H_5$ | H | $-CHCl_2$ | $-C_2H_4OC_2H_5$ |
| $CH_3$ | 6-$C_2H_5$ | H | $-CHCl_2$ | $-C_2H_4OC_2H_4OCH_3$ |
| $CH_3$ | 6-$C_2H_5$ | H | $-CHCl_2$ | $-CH_2CN$ |
| H | H | H | $-CH_2OCOCH_3$ | $-CH_3$ |
| H | H | H | $-CH_2OCOCH_3$ | $-C_2H_5$ |
| H | H | H | $-CH_2OCOCH_3$ | $-CH_2CH_2OCH_3$ |
| H | H | H | $-CH_2OCOCH_3$ | $-CH_2-CH-CH_2$ |
| H | H | H | $-CH_2OCOCH_3$ | $-CH_2-C\equiv CH$ |
| H | H | H | $-CH_2OCOCH_3$ | $-C_2H_4OC_2H_4OCH_3$ |
| H | H | H | $-CH_2OCOCH_3$ | $-CH_2CN$ |
| $CH_3$ | 6-$C_2H_5$ | H | $-CH_2-OCOCH_3$ | $-CH_3$ |

Fortsetzung

| $R^1$ | $R^2$ | $R^3$ | $R^4$ | R |
|---|---|---|---|---|
| $CH_3$ | 6-$C_2H_5$ | H | $—CH_2—OCOCH_3$ | $—C_2H_5$ |
| $CH_3$ | 6-$C_2H_5$ | H | $—CH_2—OCOCH_3$ | $—CH_2CH_2OCH_3$ |
| $CH_3$ | 6-$C_2H_5$ | H | $—CH_2—OCOCH_3$ | $—CH_2—CH{=}CH_2$ |
| $CH_3$ | 6-$C_2H_5$ | H | $—CH_2—OCOCH_3$ | $—CH_2—C{\equiv}CH$ |
| $CH_3$ | 6-$C_2H_5$ | H | $—CH_2—OCOCH_3$ | $—CH_2CH_2OC_2H_5$ |
| $CH_3$ | 6-$C_2H_5$ | H | $—CH_2—OCOCH_3$ | $—C_2H_4OC_2H_4OCH_3$ |
| $CH_3$ | 6-$C_2H_5$ | H | $—CH_2—OCOCH_3$ | $—CH_2CN$ |
| $CH_3$ | 6-$CH_3$ | H | $—CH_2—OCOCH_3$ | $—C_2H_5$ |
| $CH_3$ | 6-$CH_3$ | H | $—CH_2—OCOCH_3$ | $—CH_2CH_2OCH_3$ |
| $CH_3$ | 6-$CH_3$ | H | $—CH_2—OCOCH_3$ | $—CH_2—CH{=}CH_2$ |
| $CH_3$ | 6-$CH_3$ | H | $—CH_2—OCOCH_3$ | $—CH_2—C{\equiv}CH$ |
| $CH_3$ | 6-$CH_3$ | H | $—CH_2—OCOCH_3$ | $—CH_2CH_2OC_2H_5$ |
| $CH_3$ | 6-$CH_3$ | H | $—CH_2—OCOCH_3$ | $—C_2H_4OC_2H_4OCH_3$ |
| $CH_3$ | 6-$CH_3$ | H | $—CH_2—OCOCH_3$ | $—CH_2CN$ |
| H | H | H | 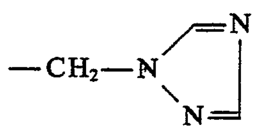 | $—CH_3$ |
| H | H | H | 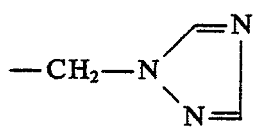 | $—C_2H_5$ |
| H | H | H | 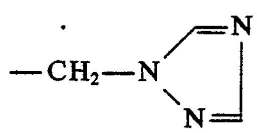 | $—C_2H_4OCH_3$ |
| H | H | H | 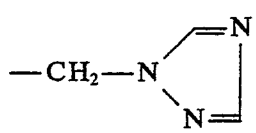 | $—CH_2—CH{=}CH_2$ |
| H | H | H | 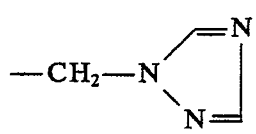 | $—CH_2—C{\equiv}CH$ |
| H | H | H | 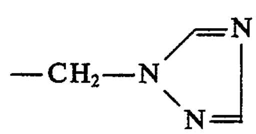 | $—C_2H_4OC_2H_5$ |

Fortsetzung

| $R^1$ | $R^2$ | $R^3$ | $R^4$ | R |
|---|---|---|---|---|
| H | H | H | 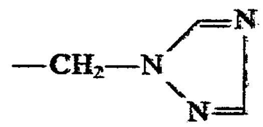 | $-C_2H_4OC_2H_4OCH_3$ |
| H | H | H | 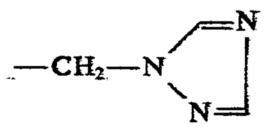 | $-CH_2CN$ |
| $CH_3$ | 6-$CH_3$ | H | 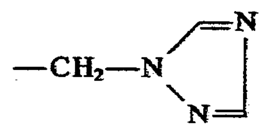 | $-C_2H_5$ |
| $CH_3$ | 6-$CH_3$ | H | 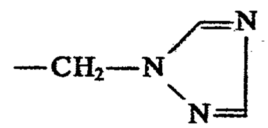 | $-C_2H_4OCH_3$ |
| $CH_3$ | 6-$CH_3$ | H | 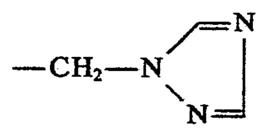 | $-CH_2-CH=CH_2$ |
| $CH_3$ | 6-$CH_3$ | H | 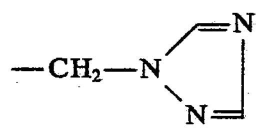 | $-CH_2-C\equiv CH$ |
| $CH_3$ | 6-$CH_3$ | H | 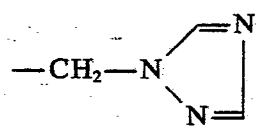 | $-C_2H_4OC_2H_5$ |
| $CH_3$ | 6-$CH_3$ | H | 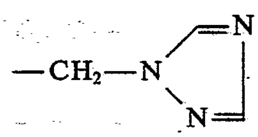 | $-C_2H_4OC_2H_4OCH_3$ |
| $CH_3$ | 6-$CH_3$ | H | 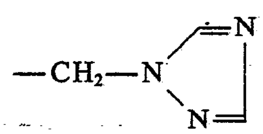 | $-CH_2CN$ |
| $CH_3$ | 6-$C_2H_5$ | H | 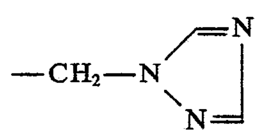 | $-CH_3$ |
| $CH_3$ | 6-$C_2H_5$ | H | 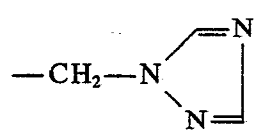 | $-C_2H_5$ |
| $CH_3$ | 6-$C_2H_5$ | H | 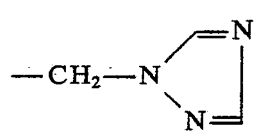 | $-C_2H_4OCH_3$ |

Fortsetzung

| $R^1$ | $R^2$ | $R^3$ | $R^4$ | R |
|---|---|---|---|---|
| $CH_3$ | 6-$C_2H_5$ | H | 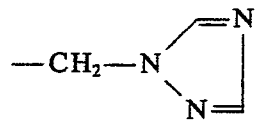 | $—CH_2—CH{=}CH_2$ |
| $CH_3$ | 6-$C_2H_5$ | H | 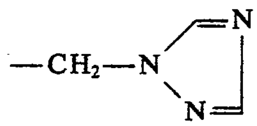 | $—CH_2—C{\equiv}CH$ |
| $CH_3$ | 6-$C_2H_5$ | H | 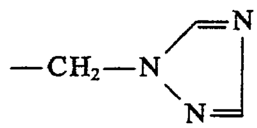 | $—C_2H_4OC_2H_5$ |
| $CH_3$ | 6-$C_2H_5$ | H | 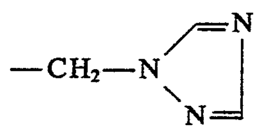 | $—C_2H_4OC_2H_4OCH_3$ |
| $CH_3$ | 6-$C_2H_5$ | H | 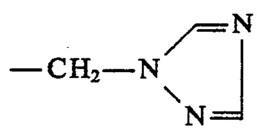 | $—CH_2CN$ |
| $CH_3$ | H | H | 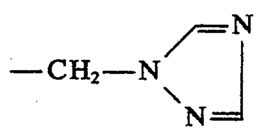 | $—CH_3$ |
| $CH_3$ | H | H | $—CHCl_2$ | $—CH_3$ |
| Cl | 6-$CH_3$ | H | 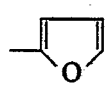 | $—CH_3$ |
| Cl | 6-$CH_3$ | H | $—CH_2OCH_3$ | $—CH_3$ |
| Cl | 6-$CH_3$ | H | 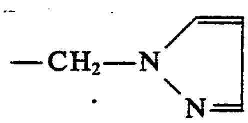 | $—CH_3$ |
| Cl | 6-$CH_3$ | H | 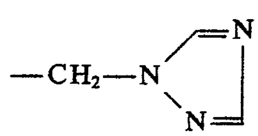 | $—CH_3$ |
| Cl | 6-$CH_3$ | H | $—CH_2Cl_2$ | $—CH_3$ |
| $C(CH_3)_3$ | H | H | 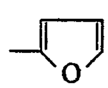 | $—CH_3$ |
| $C(CH_3)_3$ | H | H | $—CH_2OCH_3$ | $—CH_3$ |
| $C(CH_3)_3$ | H | H | 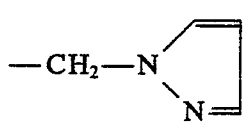 | $—CH_3$ |

Fortsetzung

| $R^1$ | $R^2$ | $R^3$ | $R^4$ | R |
|---|---|---|---|---|
| $C(CH_3)_3$ | H | H | 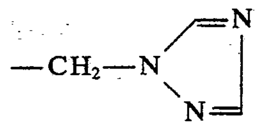 | $-CH_3$ |
| $C(CH_3)_3$ | H | H | $-CHCl_2$ | $-CH_3$ |
| $CH_3$ | 4-$CH_3$ | 6-$CH_3$ | 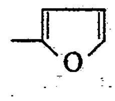 | $-CH_3$ |
| $CH_3$ | 4-$CH_3$ | 6-$CH_3$ | $-CH_2OCH_3$ | $-CH_3$ |
| $CH_3$ | 4-$CH_3$ | 6-$CH_3$ | 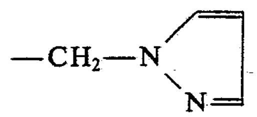 | $-CH_3$ |
| $CH_3$ | 4-$CH_3$ | 6-$CH_3$ | 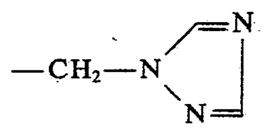 | $-CH_3$ |
| $CH_3$ | 4-$CH_3$ | 6-$CH_3$ | $-CHCl_2$ | $-CH_3$ |

Verwendet man beispielsweise N-(2,6-Xylyl)-ethylglycinmethylester und Methoxyacetylchlorid als Ausgangsstoffe, so kann der Reaktionsablauf durch das folgende Formelschema wiedergegeben werden (Verfahren a):

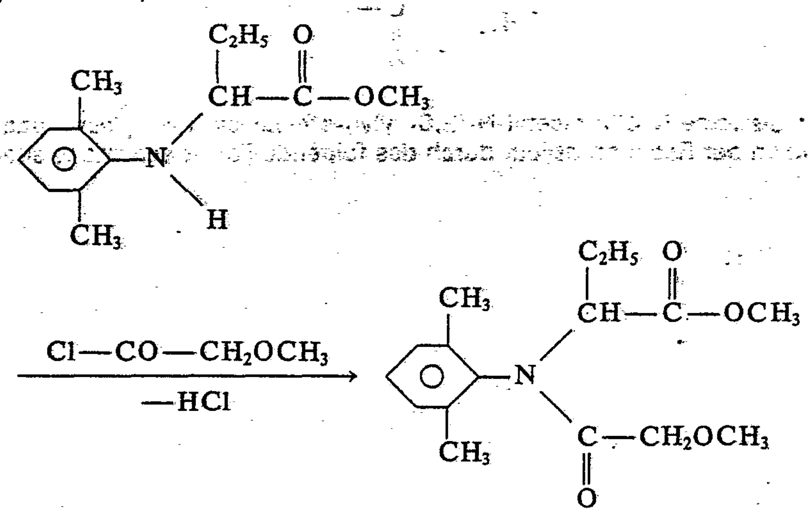


Verwendet man beispielsweise N-Dichloracetyl-N-(2,6-xylyl)-ethylglycin und Propargylalkohol als Ausgangsstoffe und DCC* als Kondensationsmittel, so kann der Reaktionsablauf durch das folgende Formelschema wiedergegeben werden (Verfahren b):

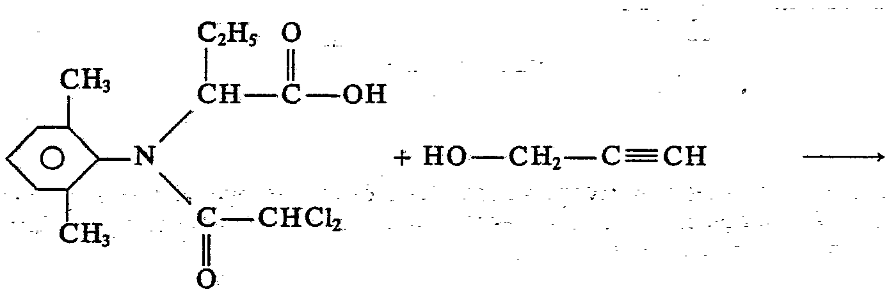

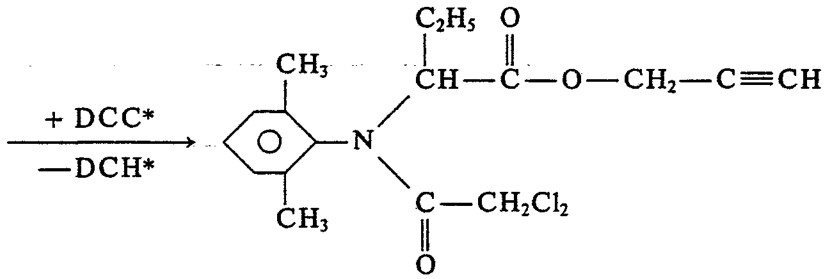


*DCC = Dicyclohexylcarbodiimid
*DCH = Dicyclohexylharnstoff

Verwendet man beispielsweise N-(2-Furoyl)-N-(2,6-xylyl)-ethyl-glycinchlorid und O-Methylglykol als Ausgangsstoffe, so kann der Reaktionsablauf durch das folgende Formelschema wiedergegeben werden (Verfahren c):

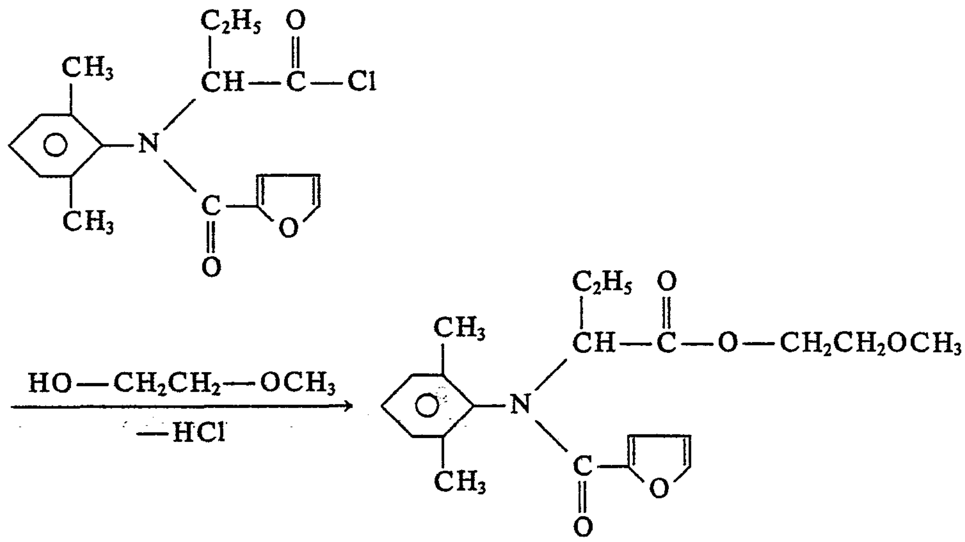


Verwendet man beispielsweise N-Chloracetyl-N-(2,6-xylyl)-ethylglycin-methylester und Imidazol als Ausgangsstoffe, so kann der Reaktionsablauf durch das folgende Formelschema wiedergegeben werden (Verfahren d):

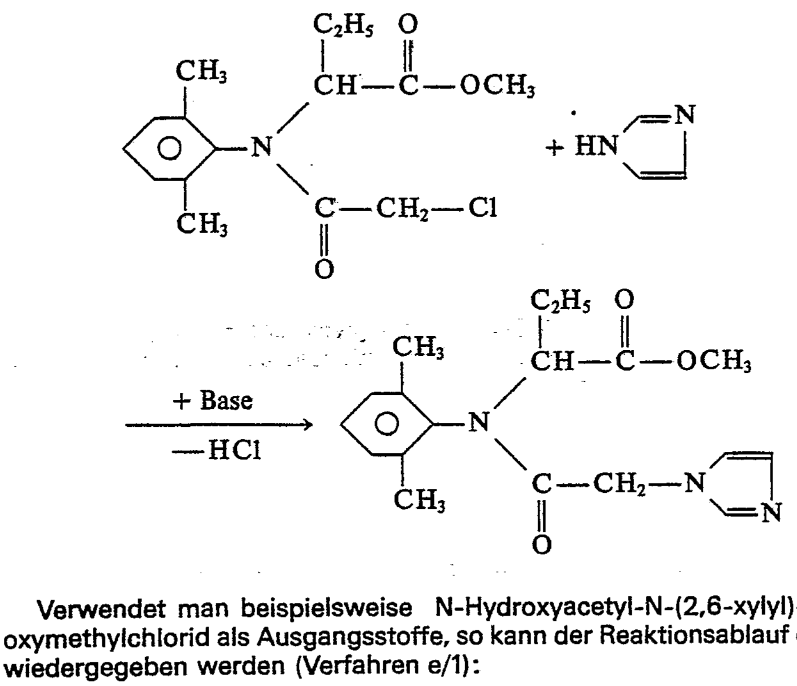


Verwendet man beispielsweise N-Hydroxyacetyl-N-(2,6-xylyl)-ethylglycin-methylester und Ethoxymethylchlorid als Ausgangsstoffe, so kann der Reaktionsablauf durch das folgende Formelschema wiedergegeben werden (Verfahren e/1):

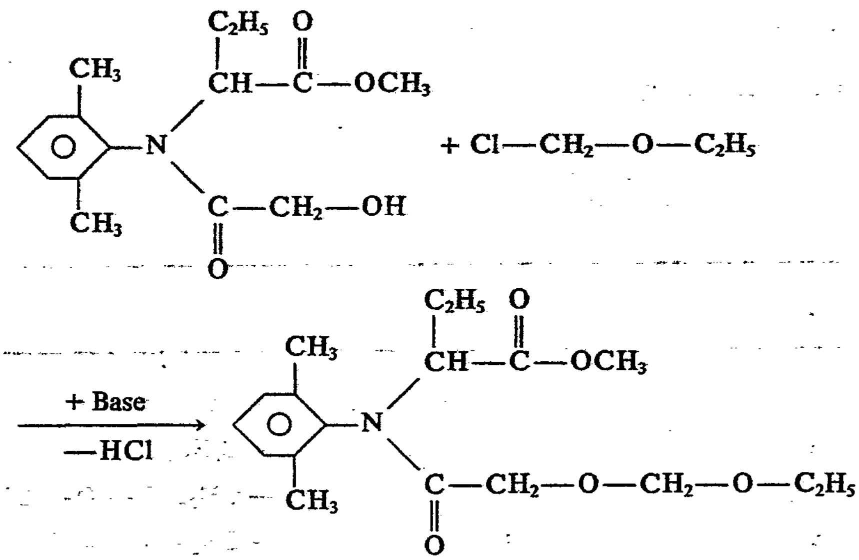


Verwendet man beispielsweise N-Hydroxyacetyl-N-(2,6-xylyl)-ethyl-glycinmethylester und 3,4-Dihydro-4H-pyran als Ausgangsstoffe, so kann der Reaktionsablauf durch das folgende Formelschema wiedergegeben werden (Verfahren e/2):

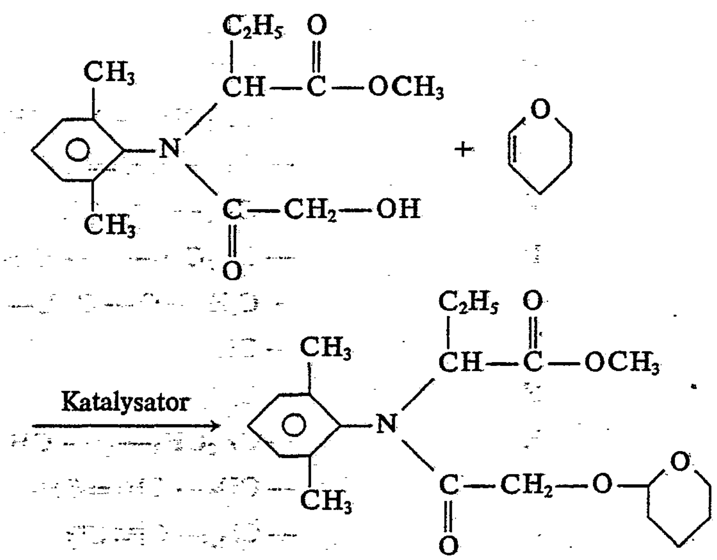


Die bei der Durchführung des erfindungsgemäßen Verfahrens (a) als Ausgangsstoffe benötigten N-Phenyl-ethylglycinester sind durch die Formel (II) allgemein definiert. In dieser Formel stehen R, $R^1$, $R^2$ und $R^3$ vorzugsweise für diejenigen Reste, die bereits im Zusammenhang mit der Beschreibung der erfindungsgemäßen Stoffe der Formel (I) vorzugsweise für diese Substituenten genannt wurden.

Die N-Phenyl-ethylglycinester der Formel (II) sind teilweise beschrieben (vergleiche US-Patentschrift 4 096 167 sowie DE-OS 2 805 525). Sie können erhalten werden, indem man z. B. entsprechende Aniline mit entsprechenden $\alpha$-Halogen-buttersäureestern in Gegenwart eines Säurebindemittels, wie beispielsweise Kaliumcarbonat, und in Gegenwart eines inerten organischen Lösungsmittels, wie beispielsweise Dimethylformamid, und gegebenenfalls in Gegenwart eines Katalysators, wie z. B. Kaliumjodid, bei Temperaturen zwischen 20 und 100° C umsetzt.

Die N-Phenyl-ethylglycinester der Formel (II) können auch erhalten werden, wenn man N-Phenyl-ethylglycine mit entsprechenden Alkoholen nach bekannten Verfahren, z. B. in Gegenwart von Bortrifluorid, verestert (vergleiche hierzu auch die Angaben für das Verfahren b).

Als Beispiele für die Ausgangsstoffe der Formel (II) seien genannt:

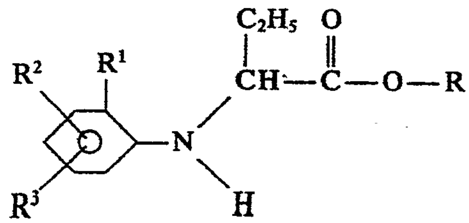

(II)

| $R^1$ | $R^2$ | $R^3$ | R |
|---|---|---|---|
| H | H | H | $-CH_3$ |
| H | H | H | $-C_2H_5$ |
| H | H | H | $-CH_2CH_2-O-CH_3$ |
| H | H | H | $-CH_2-CH=CH_2$ |
| H | H | H | $-CH_2-C\equiv CH$ |
| H | H | H | $-CH_2CH_2-O-C_2H_5$ |
| H | H | H | $-C_2H_4-O-C_2H_4-OCH_3$ |
| $CH_3$ | 6-$CH_3$ | H | $-CH_3$ |
| $CH_3$ | 6-$CH_3$ | H | $-C_2H_5$ |
| $CH_3$ | 6-$CH_3$ | H | $-CH_2CH_2-O-CH_3$ |
| $CH_3$ | 6-$CH_3$ | H | $-CH_2-CH=CH_2$ |
| $CH_3$ | 6-$CH_3$ | H | $-CH_2-C\equiv CH$ |
| $CH_3$ | 6-$CH_3$ | H | $-CH_2CH_2-O-C_2H_5$ |
| $CH_3$ | 6-$CH_3$ | H | $-C_2H_4-O-C_2H_4-OCH_3$ |
| $C_2H_5$ | 6-$C_2H_5$ | H | $-CH_3$ |
| $C_2H_5$ | 6-$C_2H_5$ | H | $-C_2H_5$ |
| $C_2H_5$ | 6-$C_2H_5$ | H | $-CH_2CH_2-O-CH_3$ |
| $C_2H_5$ | 6-$C_2H_5$ | H | $-CH_2-CH=CH_2$ |
| $C_2H_5$ | 6-$C_2H_5$ | H | $-CH_2-C\equiv CH$ |
| $C_2H_5$ | 6-$C_2H_5$ | H | $-CH_2CH_2-O-C_2H_5$ |
| $C_2H_5$ | 6-$C_2H_5$ | H | $-C_2H_4-O-C_2H_4-OCH_3$ |
| $C_2H_5$ | 6-$CH_3$ | H | $-CH_3$ |
| $C_2H_5$ | 6-$CH_3$ | H | $-C_2H_5$ |
| $C_2H_5$ | 6-$CH_3$ | H | $-CH_2CH_2-O-CH_3$ |
| $C_2H_5$ | 6-$CH_3$ | H | $-CH_2-CH=CH_2$ |
| $C_2H_5$ | 6-$CH_3$ | H | $-CH_2-C\equiv CH$ |
| $C_2H_5$ | 6-$CH_3$ | H | $-CH_2CH_2-O-C_2H_5$ |
| $C_2H_5$ | 6-$CH_3$ | H | $-C_2H_4-O-C_2H_4-OCH_3$ |
| $C(CH_3)_3$ | H | H | $-CH_3$ |
| $C(CH_3)_3$ | H | H | $-C_2H_5$ |
| $C(CH_3)_3$ | H | H | $-CH_2CH_2-O-CH_3$ |
| $C(CH_3)_3$ | H | H | $-CH_2-CH=CH_2$ |
| $C(CH_3)_3$ | H | H | $-CH_2-C\equiv CH$ |

Fortsetzung

| $R^1$ | $R^2$ | $R^3$ | R |
|---|---|---|---|
| $C(CH_3)_3$ | H | H | $-CH_2CH_2-O-C_2H_5$ |
| $C(CH_3)_3$ | H | H | $-C_2H_4-O-C_2H_4-OCH_3$ |
| $CH_3$ | 3-$CH_3$ | H | $-CH_3$ |
| $CH_3$ | 3-$CH_3$ | H | $-C_2H_5$ |
| $CH_3$ | 3-$CH_3$ | H | $-CH_2CH_2-O-CH_3$ |
| $CH_3$ | 3-$CH_3$ | H | $-CH_2-CH=CH_2$ |
| $CH_3$ | 3-$CH_3$ | H | $-CH_2-C\equiv CH$ |
| $CH_3$ | 3-$CH_3$ | H | $-CH_2CH_2-O-C_2H_5$ |
| $CH_3$ | 3-$CH_3$ | H | $-C_2H_4-O-C_2H_4-OCH_3$ |
| $C_2H_5$ | H | H | $-CH_3$ |
| $C_2H_5$ | H | H | $-C_2H_5$ |
| $C_2H_5$ | H | H | $-CH_2CH_2-O-CH_3$ |
| $C_2H_5$ | H | H | $-CH_2-CH=CH_2$ |
| $C_2H_5$ | H | H | $-CH_2-C\equiv CH$ |
| $C_2H_5$ | H | H | $-CH_2CH_2-O-C_2H_5$ |
| $C_2H_5$ | H | H | $-C_2H_4-O-C_2H_4-OCH_3$ |
| i-$C_3H_7$ | H | H | $-CH_3$ |
| i-$C_3H_7$ | H | H | $-C_2H_5$ |
| i-$C_3H_7$ | H | H | $-CH_2CH_2-O-CH_3$ |
| i-$C_3H_7$ | H | H | $-CH_2-CH=CH_2$ |
| i-$C_3H_7$ | H | H | $-CH_2-C\equiv CH$ |
| i-$C_3H_7$ | H | H | $-CH_2CH_2-O-C_2H_5$ |
| i-$C_3H_7$ | H | H | $-C_2H_4-O-C_2H_4-OCH_3$ |
| $CH_3$ | 5-$CH_3$ | H | $-CH_3$ |
| $CH_3$ | 5-$CH_3$ | H | $-C_2H_5$ |
| $CH_3$ | 5-$CH_3$ | H | $-CH_2CH_2-O-CH_3$ |
| $CH_3$ | 5-$CH_3$ | H | $-CH_2-CH=CH_2$ |
| $CH_3$ | 5-$CH_3$ | H | $-CH_2-C\equiv CH$ |
| $CH_3$ | 5-$CH_3$ | H | $-CH_2CH_2-O-C_2H_5$ |
| $CH_3$ | 5-$CH_3$ | H | $-C_2H_4-O-C_2H_4-OCH_3$ |
| Cl | 6-$CH_3$ | H | $-CH_3$ |
| Cl | 6-$CH_3$ | H | $-C_2H_5$ |
| Cl | 6-$CH_3$ | H | $-CH_2CH_2-O-CH_3$ |
| Cl | 6-$CH_3$ | H | $-CH_2-CH=CH_2$ |
| Cl | 6-$CH_3$ | H | $-CH_2-C\equiv CH$ |
| Cl | 6-$CH_3$ | H | $-CH_2CH_2-O-C_2H_5$ |
| Cl | 6-$CH_3$ | H | $-C_2H_4-O-C_2H_4-OCH_3$ |

Fortsetzung

| $R^1$ | $R^2$ | $R^3$ | R |
|---|---|---|---|
| Cl | 6-$C(CH_3)_3$ | H | $-CH_3$ |
| Cl | 6-$C(CH_3)_3$ | H | $-C_2H_5$ |
| Cl | 6-$C(CH_3)_3$ | H | $-CH_2CH_2-O-CH_3$ |
| Cl | 6-$C(CH_3)_3$ | H | $-CH_2-CH=CH_2$ |
| Cl | 6-$C(CH_3)_3$ | H | $-CH_2-C\equiv CH$ |
| Cl | 6-$C(CH_3)_3$ | H | $-CH_2CH_2-O-C_2H_5$ |
| Cl | 6-$C(CH_3)_3$ | H | $-C_2H_4-O-C_2H_4-OCH_3$ |
| $CH_3$ | 3-$CH_3$ | 6-$CH_3$ | $-CH_3$ |
| $CH_3$ | 3-$CH_3$ | 6-$CH_3$ | $-C_2H_5$ |
| $CH_3$ | 3-$CH_3$ | 6-$CH_3$ | $-CH_2CH_2-O-CH_3$ |
| $CH_3$ | 3-$CH_3$ | 6-$CH_3$ | $-CH_2-CH=CH_2$ |
| $CH_3$ | 3-$CH_3$ | 6-$CH_3$ | $-CH_2-C\equiv CH$ |
| $CH_3$ | 3-$CH_3$ | 6-$CH_3$ | $-CH_2CH_2-O-C_2H_5$ |
| $CH_3$ | 3-$CH_3$ | 6-$CH_3$ | $-C_2H_4-O-C_2H_4-OCH_3$ |
| $CH_3$ | 4-$CH_3$ | 6-$CH_3$ | $-CH_3$ |
| $CH_3$ | 4-$CH_3$ | 6-$CH_3$ | $-C_2H_5$ |
| $CH_3$ | 4-$CH_3$ | 6-$CH_3$ | $-CH_2CH_2-O-CH_3$ |
| $CH_3$ | 4-$CH_3$ | 6-$CH_3$ | $-CH_2-CH=CH_2$ |
| $CH_3$ | 4-$CH_3$ | 6-$CH_3$ | $-CH_2-C\equiv CH$ |
| $CH_3$ | 4-$CH_3$ | 6-$CH_3$ | $-CH_2CH_2-O-C_2H_5$ |
| $CH_3$ | 4-$CH_3$ | 6-$CH_3$ | $-C_2H_4-O-C_2H_4-OCH_3$ |
| i-$C_3H_7$ | 6-i-$C_3H_7$ | H | $-CH_3$ |
| i-$C_3H_7$ | 6-i-$C_3H_7$ | H | $-C_2H_5$ |
| i-$C_3H_7$ | 6-i-$C_3H_7$ | H | $-CH_2CH_2-O-CH_3$ |
| i-$C_3H_7$ | 6-i-$C_3H_7$ | H | $-CH_2-CH=CH_2$ |
| i-$C_3H_7$ | 6-i-$C_3H_7$ | H | $-CH_2-C\equiv CH$ |
| i-$C_3H_7$ | 6-i-$C_3H_7$ | H | $-CH_2CH_2-O-C_2H_5$ |
| i-$C_3H_7$ | 6-i-$C_3H_7$ | H | $-C_2H_4-O-C_2H_4-OCH_3$ |
| $OCH_3$ | 6-$CH_3$ | H | $-CH_3$ |
| $OCH_3$ | 6-$CH_3$ | H | $-C_2H_5$ |
| $OCH_3$ | 6-$CH_3$ | H | $-CH_2CH_2-O-CH_3$ |
| $OCH_3$ | 6-$CH_3$ | H | $-CH_2-CH=CH_2$ |
| $OCH_3$ | 6-$CH_3$ | H | $-CH_2-C\equiv CH$ |
| $OCH_3$ | 6-$CH_3$ | H | $-CH_2CH_2-O-C_2H_5$ |
| $OCH_3$ | 6-$CH_3$ | H | $-C_2H_4-O-C_2H_4-OCH_3$ |
| Br | 6-$CH_3$ | H | $-CH_3$ |
| Br | 6-$CH_3$ | H | $-C_2H_5$ |

Fortsetzung

| $R^1$ | $R^2$ | $R^3$ | R |
|---|---|---|---|
| Br | 6-$CH_3$ | H | $-CH_2CH_2-O-CH_3$ |
| Br | 6-$CH_3$ | H | $-CH_2-CH=CH_2$ |
| Br | 6-$CH_3$ | H | $-CH_2-C\equiv CH$ |
| Br | 6-$CH_3$ | H | $-CH_2CH_2-O-C_2H_5$ |
| Br | 6-$CH_3$ | H | $-C_2H_4-O-C_2H_4-OCH_3$ |
| $OCH_3$ | 6-$OCH_3$ | H | $-CH_3$ |
| $OCH_3$ | 6-$OCH_3$ | H | $-C_2H_5$ |
| $OCH_3$ | 6-$OCH_3$ | H | $-CH_2CH_2-O-CH_3$ |
| $OCH_3$ | 6-$OCH_3$ | H | $-CH_2-CH=CH_2$ |
| $OCH_3$ | 6-$OCH_3$ | H | $-CH_2-C\equiv CH$ |
| $OCH_3$ | 6-$OCH_3$ | H | $-CH_2CH_2-O-C_2H_5$ |
| $OCH_3$ | 6-$OCH_3$ | H | $-C_2H_4-O-C_2H_4-OCH_3$ |

Die außerdem für das erfindungsgemäße Verfahren (a) als Ausgangsstoffe zu verwendenden Säurechloride und -bromide bzw. -anhydride sind durch die Formeln (IIIa) und (IIIb) allgemein definiert. In diesen Formeln steht $R^4$ vorzugsweise für diejenigen Reste, die bereits im Zusammenhang mit der Beschreibung der erfindungsgemäßen Stoffe der Formel (I) vorzugsweise für diesen Substituenten genannt wurden.

Die Säurechloride und -bromide bzw. -anhydride der Formeln (IIIa) und (IIIb) sind allgemein bekannte Verbindungen der organischen Chemie.

Die für die Verfahrensvariante (b) als Ausgangsstoffe zu verwendenden N-Acyl-N-phenyl-ethylglycine sind durch die Formel (IV) allgemein definiert. In dieser Formel stehen $R^1$, $R^2$, $R^3$ und $R^4$ vorzugsweise für die Reste, die im Zusammenhang mit der Beschreibung der erfindungsgemäßen Stoffe der Formel (I) bereits vorzugsweise für diese Substituenten genannt wurden.

Die N-Acyl-N-phenyl-ethylglycine der Formel (IV) sind noch nicht bekannt. Sie können jedoch auf einfache Weise erhalten werden, indem man N-Phenyl-ethylglycine mit Säurechloriden der Formeln (IIIa) in Gegenwart eines Säurebindemittels, wie beispielsweise Natronlauge, und in Gegenwart eines inerten organischen Lösungsmittels, wie beispielsweise Methylenchlorid bei Temperaturen zwischen 0 und 120° C umsetzt.

Die außerdem für die Verfahrensvarianten (b) und (c) als Ausgangsstoffe zu verwendenden Alkohole sind durch die Formeln (V) allgemein definiert. In dieser Formel steht R vorzugsweise für die Reste, die im Zusammenhang mit der Beschreibung der erfindungsgemäßen Stoffe der Formel (I) bereits vorzugsweise für diese Substituenten genannt wurden.

Die Alkohole der Formel (V) sind allgemein bekannte Verbindungen der organischen Chemie.

Die für die Verfahrensvariante (c) als Ausgangsstoffe zu verwendenden N-Acyl-N-phenyl-ethylglycinchloride sind durch die Formeln (VI) allgemein definiert. In dieser Formel stehen $R^1$, $R^2$, $R^3$ und $R^4$ vorzugsweise für die Reste, die im Zusammenhang mit der Beschreibung der erfindungsgemäßen Stoffe der Formel (I) bereits vorzugsweise für diese Substituenten genannt wurden.

Die N-Acyl-N-phenyl-ethylglycinchloride der Formel (VI) sind noch nicht bekannt. Sie können jedoch auf einfache Weise erhalten werden, indem man die N-Acyl-N-phenyl-ethylglycine der Formel (IV) vorzugsweise mit Oxalylchlorid in Gegenwart von Dimethylformamid oder Pyridin umsetzt.

Die bei der Durchführung des erfindungsgemäßen Verfahrens (d) als Ausgangsstoffe benötigten N-Chloracetyl-N-phenyl-ethylglycinester sind durch die Formel (VIII) allgemein definiert. In dieser Formel stehen R, $R^1$, $R^2$ und $R^3$ vorzugsweise für diejenigen Reste, die bereits im Zusammenhang mit der Beschreibung der erfindungsgemäßen Stoffe der Formel (I) vorzugsweise für diese Substituenten genannt wurden.

Die N-Chloracetyl-N-phenyl-ethylglycinester der Formel (VII) sind teilweise beschrieben (vergleiche US-Patentschrift 4 096 167 sowie DE-OS 2 805 525). Sie können erhalten werden, indem man z. B. N-Phenyl-ethylglycinester der Formel (II) gemäß der Verfahrensvariante (a) mit Chloressigsäurechloriden umsetzt.

Die außerdem für das erfindungsgemäße Verfahren (d) noch als Ausgangsstoffe zu verwendenden Verbindungen sind durch die Formel (VIII) allgemein definiert. In dieser Formel stehen Az und $R^5$

vorzugsweise für diejenigen Reste, die bereits im Zusammenhang mit der Beschreibung der erfindungsgemäßen Stoffe der Formel (I) vorzugsweise für diese Substituenten genannt wurden. B steht vorzugsweise für Wasserstoff, Natrium und Kalium.

Die Verbindungen der Formel (VIII) sind allgemein bekannte Verbindungen der organischen Chemie.

Die bei der Durchführung des erfindungsgemäßen Verfahrens (e) als Ausgangsstoffe benötigten N-Hydroxyacetyl-N-ethylglycinester sind durch die Formel (IX) allgemein definiert. In dieser Formel stehen R, $R^1$, $R^2$ und $R^3$ vorzugsweise für diejenigen Reste, die bereits im Zusammenhang mit der Beschreibung der erfindungsgemäßen Stoffe der Formel (I) vorzugsweise für diese Substituenten genannt wurden.

Die N-Hydroxyacetyl-N-phenylethylglycin der Formel (IX) sind erfindungsgemäße Verbindungen. Sie können auch erhalten werden, indem man

f) N-Acyloxyacetyl-N-phenyl-ethylglycinester der Formel

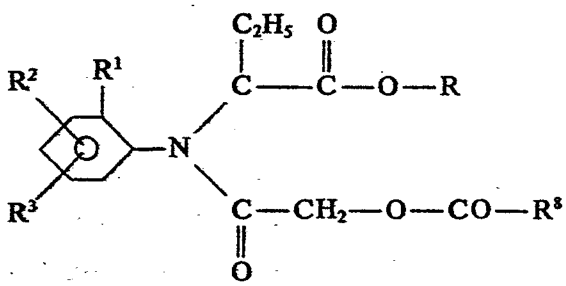

(XII)

in welcher

R, $R^1$, $R^2$ und $R^3$ die oben angegebene Bedeutung haben und
$R^8$ für Alkyl mit 1 bis 4 Kohlenstoffatomen steht,

mit Natrium- oder Kaliumhydroxid in wäßriger oder alkoholischer Lösung, z. B. in Methanol oder Ethanol, bei Temperaturen zwischen 20 und 60°C verseift und nach Ansäuern mit Verbindungen der Formel (V) in Gegenwart eines Veresterungskatalysators, wie z. B. Bortrifluorid, verestert.

Die Acyloxyacetylanilide der Formel (XII) sind ebenfalls erfindungsgemäße Verbindungen und können nach den oben beschriebenen Verfahrensvarianten (a) und (d) erhalten werden.

Die außerdem für das erfindungsgemäße Verfahren (e/1) als Ausgangsstoffe zu verwendenden Halogenide sind durch die Formel (X) allgemein definiert. In dieser Formel stehen $R^6$ und Hal für die in der Erfindungsdefinition angegebenen Reste.

Die Halogenide der Formel (X) sind allgemein bekannte Verbindungen der organischen Chemie.

Das für das erfindungsgemäße Verfahren (e/2) außerdem als Ausgangsstoff zu verwendende Dihydropyran ist ebenfalls eine bekannte Verbindung der organischen Chemie.

Als Verdünnungsmittel kommen für die erfindungsgemäße Umsetzung gemäß Verfahren (a) vorzugsweise inerte organische Lösungsmittel infrage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol und Ester, wie Essigester.

Das erfindungsgemäße Verfahren (a) kann gegebenenfalls in Gegenwart von Säurebindern [Chlor(Brom)wasserstoff-Akzeptoren] durchgeführt werden; als solche können alle üblichen Säurebindemittel verwendet werden. Hierzu gehören organische Basen, vorzugsweise tertiäre Amine, wie z. B. Triethylamin und Pyridin; ferner anorganische Basen, wie z. B. Alkalihydroxide und Alkalicarbonate. Als Katalysator kommt insbesondere Dimethylformamid in Betracht.

Die Reaktionstemperaturen können bei der Durchführung des erfindungsgemäßen Verfahrens (a) in einem größeren Bereich variiert werden. Im allgemeinen arbeitet man zwischen 0 und 120°C, vorzugsweise zwischen 20 und 100°C.

Bei der Durchführung des erfindungsgemäßen Verfahrens (a) setzt man vorzugsweise auf 1 Mol der Verbindungen der Formel (II), wenn R nicht für Hydroxyalkyl steht, 1 bis 1,5 Mol der Verbindungen der Formel (III) und gegebenenfalls 1 bis 1,5 Mol Säurebinder ein.

Wenn jedoch in diesen Ausgangsverbindungen R für Hydroxyalkyl steht, setz man auf 1 Mol dieser Verbindung 2 bis 2,5 Mol der Verbindungen der Formel (III) und gegebenenfalls 2 bis 2,5 Mol Säurebinder ein. Die Isolierung der Verbindungen der Formel (I) erfolgt in üblicher Weise.

Als Lösungsmittel kommen für die erfindungsgemäße Umsetzung gemäß Verfahrensvariante (b) vorzugsweise inerte organische Lösungsmittel infrage. Hierzu gehören vorzugsweise aromatische Kohlenwasserstoffe, wie Benzol, Toluol oder Xylol; halogenierte Kohlenwasserstoffe, wie Chloroform

oder Dichlormethan, Ester, wie Essigester; Ether wie Tetrahydrofuran, sowie die als Reaktionskomponente verwendeten Alkohole selbst.

Das erfindungsgemäße Verfahren (b) wird in Gegenwart eines Kondensationsmittels durchgeführt; als solche können alle üblichen Kondensationsmittel verwendet werden, wie insbesondere Dicyclohexylcarbodiimid (DCC), Chlorameisensäureethylester oder Benzolsulfochlorid.

Die Reaktionstemperaturen können bei der Durchführung des erfindungsgemäßen Verfahrens (b) in einem größeren Bereich variiert werden. Im allgemeinen arbeitet man zwischen 0 und 150°C, vorzugsweise zwischen 20 und 100°C bzw. bei der Siedetemperatur des verwendeten Lösungsmittels.

Bei der Durchführung des erfindungsgemäßen Verfahrens (b) setzt man vorzugsweise auf 1 Mol der Verbindungen der Formel (IV) 2 bis 8 Mol Alkohol und 1 bis 2 Mol an Kondensationsmittel ein. Die Isolierung der Verbindungen der Formel (I) erfolgt in üblicher Weise.

Als Lösungsmittel kommen für die erfindungsgemäße Umsetzung gemäß Verfahrensvariante (c) vorzugsweise inerte organische Lösungsmittel infrage. Hierzu gehören vorzugsweise die bei Verfahrensvariante (a) bereits vorzugsweise genannten Solventien. Das Verfahren kann gegebenenfalls in Gegenwart einer organischen oder anorganischen Base durchgeführt werden; bevorzugte Basen wurden bei Besprechung der Verfahrensweise (a) genannt.

Die Reaktionstemperaturen können bei der Durchführung der Verfahrensvariante (c) in einem größeren Bereich variiert werden. Im allgemeinen arbeitet man zwischen 0 und 120°C, vorzugsweise zwischen 20 und 100°C bzw. beim Siedepunkt des jeweiligen Lösungsmittels. Bei der Durchführung der Verfahrensvariante (c) arbeitet man vorzugsweise in molaren Mengen. Die Isolierung der Verbindungen der Formel (I) erfolgt in üblicher Weise.

Als Verdünnungsmittel kommen für die erfindungsgemäße Umsetzung gemäß Verfahren (d) vorzugsweise inerte organische Lösungsmittel infrage. Hierzu gehören vorzugsweise die beim Verfahren (a) bereits genannten Lösungsmittel.

Die Umsetzung nach Verfahren (d) kann gegebenenfalls in Gegenwart eines Säurebinders durchgeführt werden. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere teritäre Alkylamine, Cycloalkylamine oder Aralkylamine, beispielsweise Triethylamin, Dimethylbenzylamin; oder wie Pyridin und Diazabicyclooctan. Vorzugsweise verwendet man einen Überschuß an Azol.

Die Reaktionstemperaturen können beim Verfahren (d) in einem größeren Bereich variiert werden. Im allgemeinen arbeitet man zwischen etwa 20 und 150°C, vorzugsweise bei 60 bis 120°C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens (d) setzt man auf 1 Mol der Verbindungen der Formel (VII) vorzugsweise 1 bis 2 Mol der Verbindungen der Formel (VIII) und gegebenenfalls 1 bis 2 Mol Säurebinder ein. Die Isolierung der Verbindungen der Formel (I) erfolgt in üblicher Weise.

Als Verdünnungsmittel kommen für die erfindungsgemäße Umsetzung gemäß Verfahren (e) vorzugsweise inerte organische Lösungsmittel infrage. Hierzu gehören vorzugsweise die beim Verfahren (a) bereits genannten Lösungsmittel.

Die Umsetzung nach Verfahren (e/1) kann gegebenenfalls in Gegenwart eines Säurebinders durchgeführt werden. Man kann alle üblicherweise verwendbaren anorganischen oder organischen Säurebinder zugeben. Hierzu gehören vorzugsweise die beim Verfahren (a) bereits genannten Verbindungen.

Die Reaktionstemperaturen können beim Verfahren (e/1) in einem größeren Bereich variiert werden. Im allgemeinen arbeitet man zwischen 20 und 150°C, vorzugsweise bei der Siedetemperatur des Lösungsmittels, beispielsweise zwischen 60 und 100°C.

Bei der Durchführung des erfindungsgemäßen Verfahrens (e/1) setzt man auf 1 Mol der Verbindungen der Formel (IX), gegebenenfalls nach Zugabe von 1 bis 2 Mol einer starken Base, wie z. B. eines Alkalihydrids, 1 Mol Halogenid der Formel (X) und gegebenenfalls 1 bis 2 Mol Säurebinder ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt und in üblicher Weise aufgearbeitet.

Nach einer bevorzugten Ausführungsform des Verfahrens (e/1) wird zweckmäßigerweise so verfahren, daß man von einem Hydroxyacetanilid der Formel (IX) ausgeht, letzteres in einem geeigneten inerten Lösungsmittel mittels Alkalimetall-hydrid oder -bromid in das Alkalimetall-alkanolat überführt und letzteres ohne Isolierung sofort mit einem Halogenid der Formel (X) umsetzt, wobei unter Austritt von Alkalihalogenid die erfindungsgemäßen Verbindungen der Formel (I) in einem Arbeitsgang erhalten werden.

Die Umsetzung nach Verfahren (e/2) kann gegebenenfalls in Gegenwart eines Katalysators durchgeführt werden. Hierzu verwendet man vorzugsweise Chlorwasserstoff (vgl. J. Am. Chem. Soc. 69, 2246 [1947], ibd. 70, 4187 [1948]).

Die Reaktionstemperaturen können beim Verfahren (e/2) in einem größeren Bereich variiert werden. Im allgemeinen arbeitet man zwischen 0 und 100°C, vorzugsweise zwischen 20 und 60°C.

Bei der Durchführung des erfindungsgemäßen Verfahrens (e/2) arbeitet man vorzugsweise in

molaren Mengen. Die Isolierung der Verbindungen der Formel (I) erfolgt in üblicher Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Oomyceten, z. B. gegen den Erreger der Kraut- und Braunfäule der Tomate und Kartoffel (Phytophthora infestans) eingesetzt werden. Besonders hervorzuheben ist, daß die erfindungsgemäßen Wirkstoffe nicht nur eine protektive, sondern auch eine kurativ/eradikative Wirkung entfalten. Außerdem besitzen sie systematische Eigenschaften. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z.yB. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

In den nachfolgenden Beispielen werden die nachstehend angegebenen Verbindungen als Vergleichssubstanzen eingesetzt:

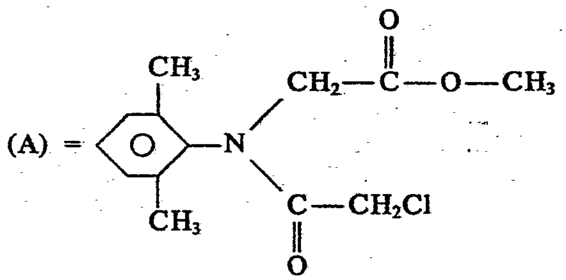

N-Chloracetyl-N-(2,6-xylyl)-glycinmethylester

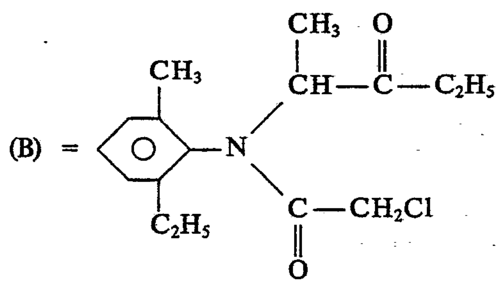

N-Chloracetyl-N-(2-ethyl-6-methylphenyl)-alaninmethylester

## Beispiel A

### Phytophthora-Test (Tomaten)/Protektiv

Lösungsmittel: 4,7 Gewichtsteile Aceton
Emulgator: 0,3 Gewichtsteile Alkyl-aryl-polyglykolether
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20° C und einer relativen Luftfeuchtigkeit von 70% im Gewächshaus. Anschließend werden die Tomatenpflanzen mit einer wäßrigen Sporensuspension von Phytophthora infestans inokuliert. Die Pflanzen werden in eine Feuchtkammer mit einer 100%igen Luftfeuchtigkeit und einer Temperatur von 18 bis 20° C gebracht.

Nach 5 Tagen wird der Befall der Tomatenpflanzen bestimmt. Die erhaltenen Boniturwerte werden auf Prozent Befall umgerechnet. 0% bedeutet keinen Befall, 100% bedeutet, daß die Pflanzen vollständig befallen sind.

In diesem Test zeigen z. B. folgende Verbindungen eine sehr gute Wirkung, die derjenigen der aus dem Stand der Technik bekannten Verbindungen (A) und (B) deutlich überlegen ist:

Verbindungen gemäß Herstellungsbeispielen: 12, 1 und 10.

Beispiel B

Phytophthora-Test (Tomaten)/Systemisch

Lösungsmittel: 4,7 Gewichtsteile Aceton
Dispergiermittel: 0,3 Gewichtsteile Alkyl-aryl-polyglykolether
Wasser: 95,0 Gewichtsteile

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.

In Einheitserde angezogene Tomatenpflanzen mit 2 bis 4 Laubblättern werden mit 10 ml der Gießflüssigkeit in der angegebenen Wirkstoffkonzentration, bezogen auf 100 ml Erde, gegossen.

Die so behandelten Pflanzen werden nach der Behandlung mit einer wäßrigen Sporensuspension von Phytophthora infestans inokuliert. Die Pflanzen werden in eine Feuchtkammer mit einer Luftfeuchtigkeit von 100% und einer Temperatur von 18 bis 20°C gebracht. Nach 5 Tagen wird der Befall der Tomatenpflanzen bestimmt. Die so erhaltenen Boniturwerte werden auf Prozent Befall umgerechnet. 0% bedeutet keinen Befall, 100% bedeutet, daß die Pflanzen vollständig befallen sind.

In diesem Test zeigen z. B. folgende Verbindungen eine sehr gute Wirkung, die derjenigen der aus dem Stand der Technik bekannten Verbindung (B) deutlich überlegen ist:

Verbindungen gemäß Herstellungsbeispielen 12, 1, 2, 8 und 10.

## Herstellungsbeispiele

### Beispiel 1

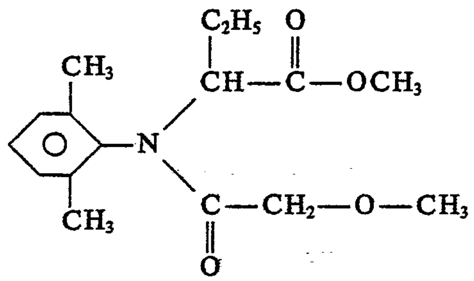

(Verfahren a)

In eine Lösung von 18,4 g (0,0816 Mol) N-(2,6-Xylyl)-ethylglycinmethylester und einigen Tropfen Dimethylformamid in 80 ml Toluol tropft man bei Raumtemperatur 13,3 g (0,122 Mol) Methoxyacetylchlorid und rührt 3 Stunden bei 80°C. Nach Abkühlung wird die Lösung mit Essigester verdünnt, mit verdünnter Salzsäure, Wasser und Natriumhydrogencarbonat-Lösung gewaschen, über Natriumsulfat getrocknet und eingedampft. Der Rückstand wird aus Ligroin kristallisiert. Man erhält 10,4 g (43,5% der Theorie) N-Methoxyacytyl-N-(2,6-xylyl)-ethylglycin-methylester vom Schmelzpunkt 96–97°C.

### Herstellung des Ausgangsproduktes

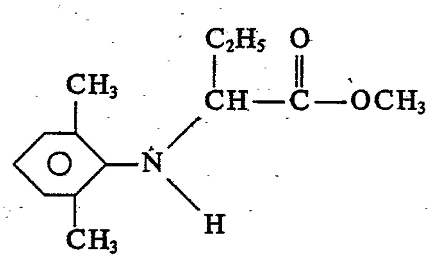

Eine Mischung aus 387,3 g (2,1 Mol) 2-Brombuttersäuremethylester, 127,0 g (1,05 Mol) 2,6-Xylidin, 121,8 g (2,1 Mol) Kaliumfluorid und 100 ml Dimethylformamid wird unter Stickstoff 39 Stunden bei 100°C gerührt. Nach Abkühlung wird filtriert, das Filtrat in Wasser gegossen, das abgeschiedene Öl mit Methylenchlorid extrahiert, der Extrakt mit Wasser gewaschen, über Natriumsulfat getrocknet und eingedampft. Der Rückstand wird über eine Kolonne im Vakuum fraktioniert. Man erhält 112,0 g (48,3% der Theorie) N-(2,6-Xylyl)-ethylglycinmethylester vom Siedepunkt 100–110°C/0,9 mbar.

Beispiel 2

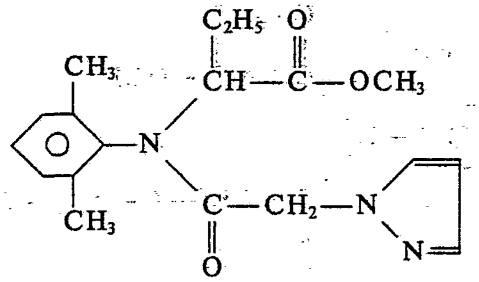

(Verfahren a)

In eine unter Rückfluß siedende Lösung von 11,1 g (0,05 Mol) N-(2,6-Xylyl)-ethylglycinmethylester, 7,9 g (0,1 Mol) Pyridin und 1,2 g (0,01 Mol) 4-Dimethylaminopyridin gibt man in 35 Minuten 18,4 g Pyrazol-1-yl-acetylchlorid-hydrochlorid (hergestellt aus 0,1 Mol Pyrazol-1-yl-essigsäure) und erhitzt 22 Stunden unter Rückfluß. Das Reaktionsgemisch wird eingedampft, der Rückstand zwischen Essigester und 1-molarer Citronensäure verteilt, die organische Phase mit 5%iger Natronlauge und Wasser gewaschen, über Natriumsulfat getrocknet und eingedampft. Der Rückstand wird aus Toluol/Petrolether (1 : 4) kristallisiert. Man erhält 11,3 g (68,7% der Theorie) N-(Pyrazol-1-yl-acetyl)-N-(2,6-xylyl)-ethylglycin-methylester vom Schmelzpunkt 113 – 115° C.

Herstellung des Ausgangsproduktes

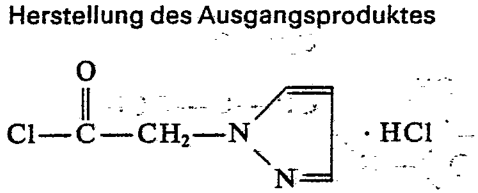

Zu einer Suspension von 12,6 g (0,1 Mol) Pyrazol-1-yl-essigsäure in 40 ml Toluol gibt man einen Tropfen Dimethylformamid und tropft bei 25 – 30° C (Eiskühlung) 19,05 g (0,15 Mol) Oxalylchlorid zu. Nach 17 Stunden Rühren bei Raumtemperatur wird abgesaugt und der Rückstand zweimal mit Toluol abgedampft. Man erhält 18,4 g rohres Pyrazol-1-yl-acetylchlorid-hydrochlorid, das ohne Reinigung weiter eingesetzt wird.

Beispiel 3

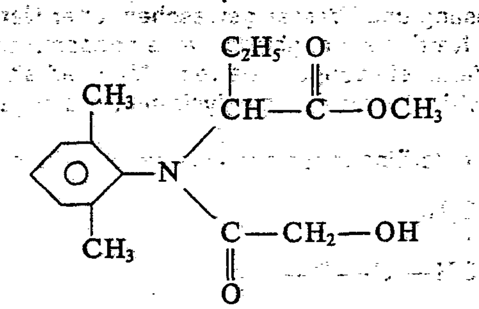

(Verfahren f)

In eine Lösung von 61,1 g (0,19 Mol) N-Acetoxyacetyl-N-(2,6-xylyl)-ethylglycin-methylester in 200 ml Methanol tropft man bei Raumtemperatur in 15 Minuten eine Lösung von 21,3 g (0,38 Mol) pulverisiertem Kaliumhydroxid in 200 ml Methanol, rührt 1 Stunde bei Raumtemperatur und 3,5 Stunden bei 50° C. Die Reaktionslösung wird eingedampft, die wäßrige Lösung des Rückstandes mit Ether ausgeschüttelt, mit halbkonzentrierter Salzsäure angesäuert und mit Essigester extrahiert. Den Extrakt trocknet man über Natriumsulfat und dampft ein.

Der Rückstand wird in 180 ml Methanol gelöst und nach Zusatz von 81,0 g (0,571 Mol) Bortrifluorid-Etherat (48%ig) 17 Stunden unter Rückfluß erhitzt. Die Reaktionslösung gießt man unter Eiskühlung in 1000 ml 10%ige Natriumhydrogencarbonat-Lösung, extrahiert mehrmals mit Methylenchlorid, wäscht den Extrakt mit Wasser, trocknet über Natriumsulfat und dampft ein. Man erhält 48,1 g (90,6% der Theorie) N-Hydroxyacetyl-N-(2,6-xylyl)-ethylglycinmethylester vom Schmelzpunkt 101 – 102° C.

Beispiel 4

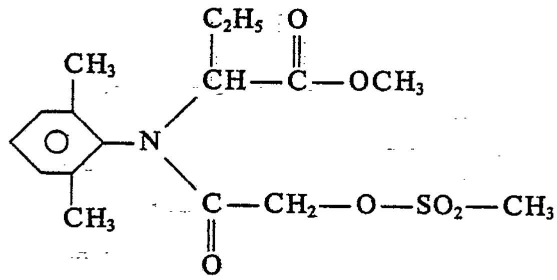

(Verfahren e/1)

In eine Lösung von 13,95 g (0,05 Mol) N-Hydroxyacetyl-N-(2,6-xylyl)-ethylglycin-methylester (Beispiel 3) und 12,3 g (0,11 Mol) 1,4-Diazabicyclo[2,2,2]octan in 150 ml Methylenchlorid tropft man bei 20–25°C (Eiskühlung) 11,5 g (0,1 Mol) Methansulfonsäurechlorid und rührt 17 Stunden bei Raumtemperatur. Die Reaktionslösung wird mit Wasser, mit 10%igem wäßrigem Pyridin, mit verdünnter Salzsäure und Wasser gewaschen, über Natriumsulfat getrocknet und eingedampft. Der Rückstand wird aus Ligroin/Toluol (7 : 2) kristallisiert. Man erhält 13,9 g (78,8% der Theorie) N-Methansulfonyl-oxyacetyl-N-(2,6-xylyl)-ethylglycinmethylester vom Schmelzpunkt 92°C.

Beispiel 5

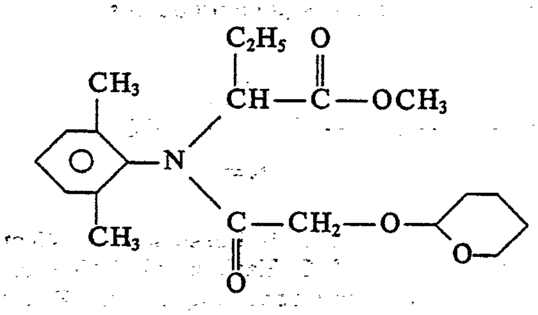

(Verfahren e/2)

Man versetzt eine Mischung aus 11,2 g (0,04 Mol) N-Hydroxyacetyl-N-(2,6-xylyl)-ethylglycinmethylester (Beispiel 3) und 13,8 g (0,16 Mol) 3,4-Dihydro-2H-pyran mit 4 ml 1-molarem etherischem Chlorwasserstoff und rührt 23 Stunden bei Raumtemperatur. Nach Verdünnen mit Essigester wird mit Natriumhydrogencarbonat-Lösung und Wasser gewaschen, über Natriumhydrogencarbonat-Lösung und Wasser gewaschen, über Natriumsulfat getrocknet, eingedampft und der ölige Rückstand an der Ölpumpe von flüchtigen Verunreinigungen befreit. Man erhält 14,4 g (99,2% der Theorie) N-Tetrahydro-pyranyloxyacetyl-N-(2,6-xylyl)-ethylglycin-methylester als Öl mit dem Brechungsindex $n_D^{20}$ =1,4959.

In analoger Weise werden die Verbindungen der allgemeinen Formel

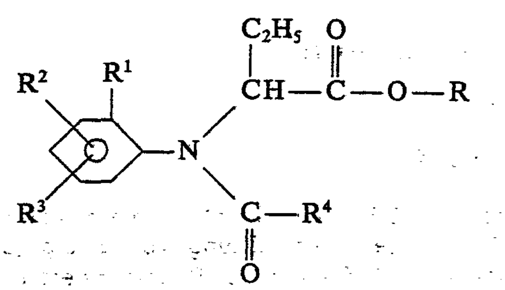

(I)

erhalten.

| Beispiel Nr. | $R^1$ | $R^2$ | $R^3$ | $R^4$ | R | Schmelzpunkt (°C) bzw. Brechungsindex |
|---|---|---|---|---|---|---|
| 6 | $CH_3$ | H | H | 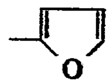 | $CH_3$ | $n_D^{21,5}$ : 1,5417 |
| 7 | $CH_3$ | H | H | $-CH_2-O-CH_3$ | $CH_3$ | $n_D^{21,5}$ : 1,5037 |
| 8 | $CH_3$ | 6-$CH_3$ | H | $-CH_2-O-COCH_3$ | $CH_3$ | 79–80 |
| 9 | $CH_3$ | 6-$CH_3$ | H | $-CHCl_2$ | $CH_3$ | 101–02 |
| 10 | $CH_3$ | 6-$CH_3$ | H | 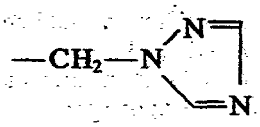 | $CH_3$ | 141–43 |
| 11 | $CH_3$ | 6-$CH_3$ | H | $-OC_2H_5$ | $CH_3$ | $n_D^{21,5}$ : 1,4865 |
| 12 | $CH_3$ | 6-$CH_3$ | H | 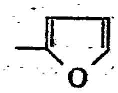 | $CH_3$ | 107 |
| 13 | $CH_3$ | 6-$CH_3$ | H | 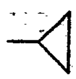 | $CH_3$ | 66–68 |
| 14 | $CH_3$ | 6-$CH_3$ | H | $-CH_2SCH_3$ | $CH_3$ | $n_D^{21}$ : 1,5470 |

**Patentansprüche**

1. N,N-disubstituierte Ethylglycinester der allgemeinen Formel

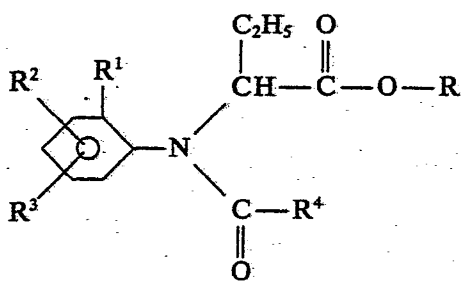

(I)

in welcher

R für Alkyl, Hydroxyalkyl, Cyanalkyl, Alkenyl, Alkinyl, Halogenalkyl, Cycloalkyl, Cycloalkylalkyl, Alkoxyalkyl, Alkoxyalkoxyalkyl, Acyloxyalkyl, Alkylthioalkyl, Alkylsulfinylalkyl, Alkylsulfonylalkyl, Dialkylaminoalkyl oder gegebenenfalls substituiertes Aralkyl steht,
$R^1$ für Wasserstoff, Alkyl, Alkoxy oder Halogen steht,
$R^2$ für Wasserstoff, Alkyl, Alkoxy oder Halogen steht,
$R^3$ für Wasserstoff oder Alkyl steht,
$R^4$ für Furyl, Tetrahydrofuryl, Thienyl, Tetrahydrothienyl; gegebenenfalls durch Alkyl substituiertes Isoxazolyl; Hydroxyalkyl; gegebenenfalls durch Cyano oder Thiocyano substituiertes Alkyl, Alkenyl, und Alkinyl; Cycloalkyl; Dihalogenalkyl; sowie die Gruppierungen

$-CH_2-Az$ $\quad -CH_2-OR^5$ $\quad -CH_2-SR^5$ $\quad -OR^5$ $\quad -SR^5$

$-CH_2-OSO_2R^5$ $\quad -CH_2OCOR^5$ und $-CH_2-O-$ 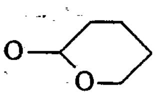

steht, wobei

$R^5$ für gegebenenfalls substituiertes Alkyl, Alkenyl, Alkinyl und Alkoxyalkyl steht, und
Az für Pyrazol-1-yl, 1,2,4-Triazol-1-yl und Imidazol-1-yl steht.

2. Verfahren zur Herstellung von N,N-disubstituierten Ethyl-glycinestern der Formel (I)

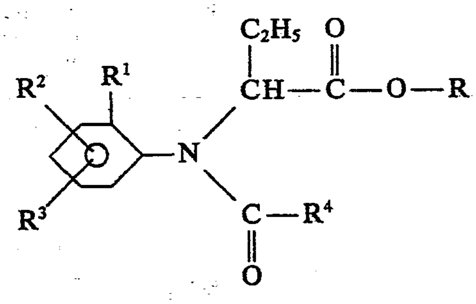

(I)

in welcher

R für Alkyl, Hydroxyalkyl, Cyanalkyl, Alkenyl, Alkinyl, Halogenalkyl, Cycloalkyl, Cycloalkylalkyl, Alkoxyalkyl, Alkoxyalkoxyalkyl, Acyloxyalkyl, Alkylthioalkyl, Alkylsulfinylalkyl, Alkylsulfonylalkyl, Dialkylaminoalkyl oder gegebenenfalls substituiertes Aralkyl steht,
$R^1$ für Wasserstoff, Alkyl, Alkoxy oder Halogen steht,
$R^2$ für Wasserstoff, Alkyl, Alkoxy oder Halogen steht,
$R^3$ für Wasserstoff oder Alkyl steht,
$R^4$ für Furyl, Tetrahydrofuryl, Thienyl, Tetrahydrothienyl; gegebenenfalls durch Alkyl substituiertes Isoxazolyl; Hydroxyalkyl; gegebenenfalls durch Cyano oder Thiocyano substituiertes Alkyl, Alkenyl und Alkinyl; Cycloalkyl; Dihalogenalkyl; sowie die Gruppierungen

$-CH_2-Az$ $-CH_2-OR^5$ $-CH_2-SR^5$ $-OR^5$ $-SR^5$

$-CH_2-OSO_2R^5$ $-CH_2OCOR^5$ und $-CH_2-O-$ 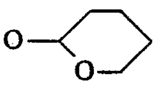

steht, wobei

$R^5$ für gegebenenfalls substituiertes Alkyl, Alkenyl, Alkinyl und Alkoxyalkyl steht, und
Az für Pyrazol-1-yl, 1,2,4-Triazol-1-yl und Imidazol-1-yl steht,

dadurch gekennzeichnet, daß man

a) N-Phenyl-ethylglycinester der Formel

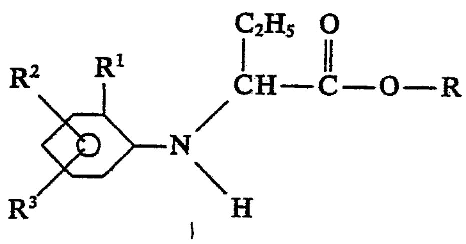

(II)

in welcher

R, $R^1$, $R^2$ und $R^3$ die bei Formel (I) angegebene Bedeutung haben,

mit Säurechloriden oder -bromiden bzw. -anhydriden der Formeln

$$R^4-\underset{\underset{O}{\|}}{C}-Cl(Br) \qquad \text{(IIIa)}$$

bzw.

$$\left(R^4-\underset{\underset{O}{\|}}{C}-\right)_2 O \qquad \text{(IIIb)}$$

in welchen

$R^4$ die bei Formel (I) angegebene Bedeutung hat,

in Gegenwart eines Verdünnungsmittels und gegebenenfalls in Gegenwart eines Säurebindemittels umsetzt, oder

b) N-Acyl-N-phenyl-ethylglycine der Formel

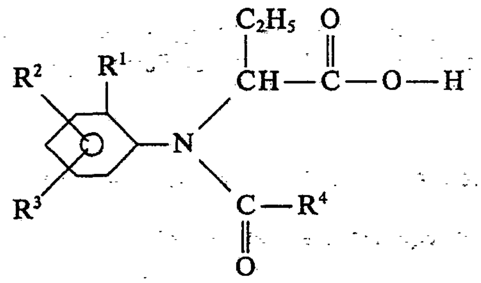

(IV)

in welcher

$R^1$, $R^2$, $R^3$ und $R^4$ die bei Formel (I) angegebene Bedeutung haben,

mit Alkoholen der Formel

R–O–H (V)

in welcher

R die bei Formel (I) angegebene Bedeutung hat,

in Gegenwart eines Kondensationsmittels und gegebenenfalls in Gegenwart eines Verdünnungsmittels umsetzt, oder

c) N-Acyl-N-phenyl-ethylglycinchloride der Formel

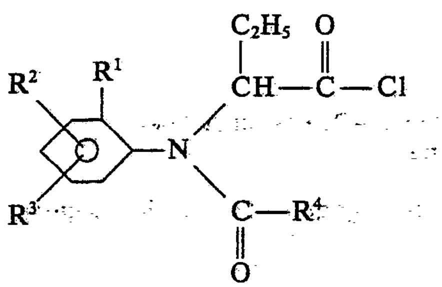

(VI)

in welcher

$R^1$, $R^2$, $R^3$ und $R^4$ die bei Formel (I) angegebene Bedeutung haben,

mit Alkoholen der Formel (V) in Gegenwart eines Verdünnungsmittels und gegebenenfalls in Gegenwart einer Base umsetzt; oder zur Darstellung einzelner Verbindungen der Formel (I)

d) N-Halogenacetyl-N-phenyl-ethylglycinester der Formel

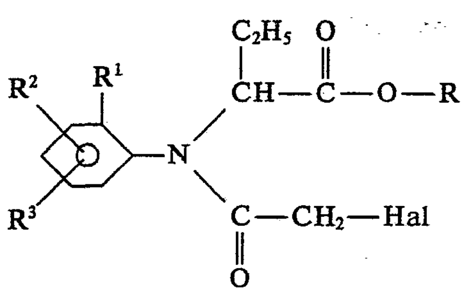

(VII)

in welcher

R, $R^1$, $R^2$ und $R^3$ die bei Formel (I) angegebene Bedeutung haben und
Hal für Chlor, Brom oder Jod steht,

mit Verbindungen der Formel

B–X (VIII)

in welcher

X für Az, Cyano, Thiocyano, Alkylcarbonyloxy mit 1 bis 4 Kohlenstoffatomen im Alkylteil und die Gruppierung $-OR^5$ oder $-SR^5$ steht, wobei Az und $R^5$ die bei Formel (I) angegebene Bedeutung haben, und
B für Wasserstoff oder ein Alkalimetall steht,

in Gegenwart eines Verdünnungsmittels und gegebenenfalls in Gegenwart eines Säurebinders umsetzt, oder

e) erfindungsgemäße N-Hydroxyacetyl-N-phenyl-ethylglycinester der Formel

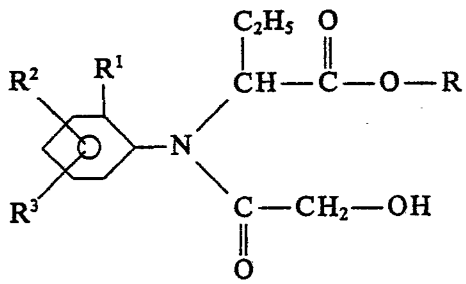

(IX)

in welcher

R, $R^1$, $R^2$ und $R^3$ die bei Formel (I) angegebene Bedeutung haben,

(1) gegebenenfalls nach Aktivierung mittels Alkalimetall mit Halogeniden der Formel

$Hal-R^6$ (X)

in welcher
Hal die oben unter d) angegebene Bedeutung hat, und
$R^6$ für den Rest $R^5$ sowie die Gruppen $-SO_2R^5$ und $-COR^5$ steht, wobei
$R^5$ die bei Formel (I) angegebene Bedeutung hat,

in Gegenwart eines Verdünnungsmittels und gegebenenfalls in Gegenwart eines Säurebinders umsetzt, oder

(2) mit Dihydropyran der Formel

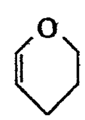

(XI)

in Gegenwart eines Verdünnungsmittels und gegebenenfalls in Gegenwart eines Katalysators umsetzt, oder

f) N-Acyloxyacetyl-N-phenyl-ethylglycinester der Formel

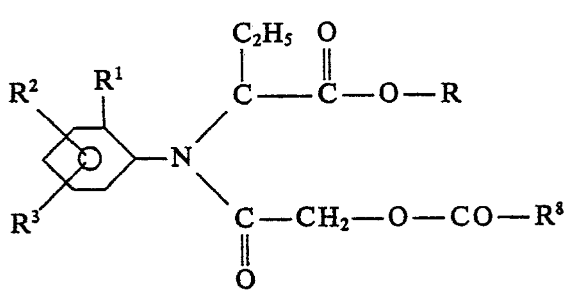

(XII)

in welcher
R, $R^1$, $R^2$ und $R^3$ die bei Formel (I) angegebene Bedeutung haben und
$R^8$ für Alkyl mit 1 bis 4 Kohlenstoffatomen steht,
mit Natrium- oder Kaliumhydroxid in wäßriger oder alkoholischer Lösung, z. B. in Methanol

oder Ethanol, bei Temperaturen zwischen 20 und 60°C verseift und nach Ansäuern mit Verbindungen der Formel (V) in Gegenwart eines Veresterungskatalysators, wie z. B. Bortrifluorid, verestert.

3. Fungizide Mittel, gekennzeichnet durch einen Gehalt an mindestens einem N,N-disubstituierten Ethylglycinester der Formel I.

4. Verfahren zur Bekämpfung von Pilzen, dadurch gekennzeichnet, daß man N,N-disubstituierte Ethylglycinester der Formel I auf Pilze oder ihren Lebensraum einwirken läßt.

5. Verwendung von N,N-disubstituierten Ethylglycinestern der Formel I zur Bekämpfung von Pilzen.

6. Verfahren zur Herstellung von fungiziden Mitteln, dadurch gekennzeichnet, daß man N,N-disubstituierte Ethylglycinester der Formel I mit Streckmitteln und/oder oberflächenaktiven Mitteln vermischt.

## Claims

1. N,N-Disubstituted ethylglycine esters of the general formula

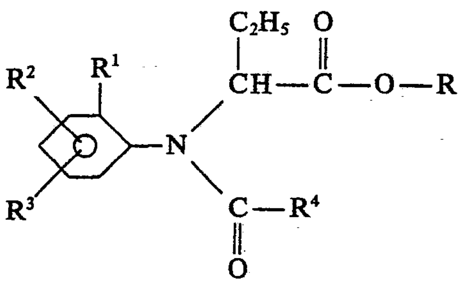

(I)

in which

R represents alkyl, hydroxyalkyl, cyanoalkyl, alkenyl, alkinyl, halogenoalkyl, cycloalkyl, cycloalkylalkyl, alkoxyalkyl, alkoxyalkoxyalkyl, acyloxyalkyl, alkylthioalkyl, alkylsulphinylalkyl, alkylsulphonylalkyl, dialkylaminoalkyl or optionally substituted aralkyl,
$R^1$ represents hydrogen, alkyl, alkoxy or halogen,
$R^2$ represents hydrogen, alkyl, alkoxy or halogen,
$R^3$ represents hydrogen or alkyl,
$R^4$ represents furyl, tetrahydrofuryl, thienyl or tetrahydrothienyl; isoxazolyl which is optionally substituted by alkyl; hydroxyalkyl; alkyl, alkenyl or alkinyl which is optionally substituted by cyano or thiocyano; cycloalkyl; dihalogenoalkyl; or the grouping

$-CH_2-Az$  $-CH_2-OR^5$  $-CH_2-SR^5$  $-OR^5$  $-SR^5$

$-CH_2-OSO_2R^5$  $-CH_2OCOR^5$  or 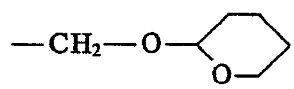

wherein

$R^5$ represents optionally substituted alkyl, alkenyl, alkinyl or alkoxyalkyl and
Az represents pyrazol-1-yl, 1,2,4-triazol-1-yl or imidazol-1-yl.

2. Process for the preparation of N,N-disubstituted ethylglycine esters of the formula (I)

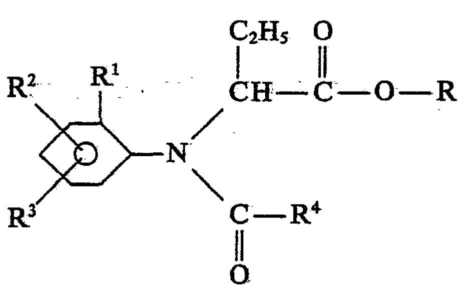

(I)

in which

R represents alkyl, hydroxyalkyl, cyanoalkyl, alkenyl, alkinyl, halogenoalkyl, cycloalkyl, cycloalkylalkyl, alkoxyalkyl, alkoxyalkoxyalkyl, acyloxyalkyl, alkylthioalkyl, alkylsulphinylalkyl, alkylsulphonylalkyl, dialkylaminoalkyl or optionally substituted aralkyl,

$R^1$ represents hydrogen, alkyl, alkoxy or halogen,

$R^2$ represents hydrogen, alkyl, alkoxy or halogen,

$R^3$ represents hydrogen or alkyl,

$R^4$ represents furyl, tetrahydrofuryl, thienyl or tetrahydrothienyl; isoxazolyl which is optionally substituted by alkyl; hydroxyalkyl; alkyl, alkenyl or alkinyl which is optionally substituted by cyano or thiocyano; cycloalkyl; dihalogenoalkyl; or the grouping

$-CH_2-Az$ $\quad -CH_2-OR^5$ $\quad -CH_2-SR^5$ $\quad -OR^5$ $\quad -SR^5$

$-CH_2-OSO_2R^5-CH_2OCOR^5$ or 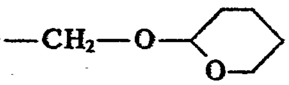

wherein

$R^5$ represents optionally substituted alkyl, alkenyl, alkinyl or alkoxyalkyl and

Az represents pyrazol-1-yl, 1,2,4-triazol-1-yl or imidazol-1-yl,

characterised in that

a) N-phenyl-ethylglycine esters of the formula

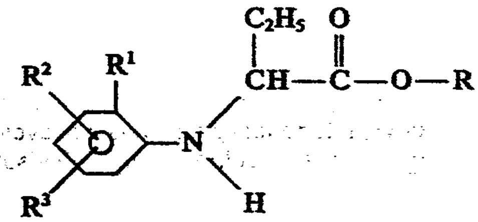

(II)

in which

R, $R^1$, $R^2$ and $R^3$ have the meaning indicated under formula (I),

are reacted with acid chlorides, bromides or anhydrides of the formulae

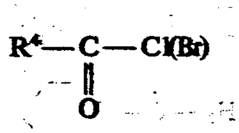

(IIIa)

or

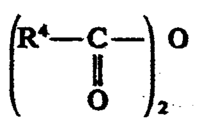

(IIIb)

in which

$R^4$ has the meaning indicated under formula (I),

in the presence of a diluent and if appropriate in the presence of an acid-binding agent, or

b) N-acyl-N-phenyl-ethylglycines of the formula

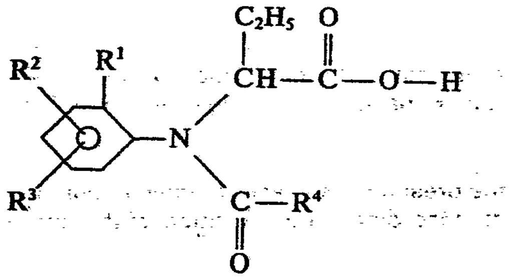

(IV)

in which

$R^1$, $R^2$, $R^3$ and $R^4$ have the meaning indicated under formula (I)

are reacted with alcohols of the formula

R–O–H (V)

in which

R has the meaning indicated under formula (I),

in the presence of a condensing agent and if appropriate in the presence of a diluent, or

c) N-acyl-N-phenyl-ethylglycine chlorides of the formula

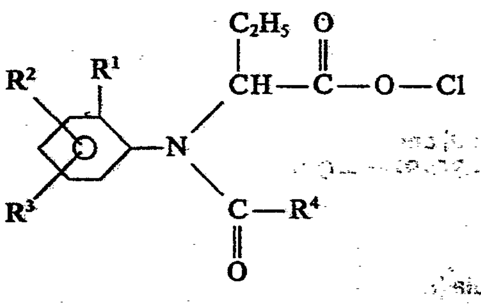

(VI)

in which

$R^1$, $R^2$, $R^3$ and $R^4$ have the meaning indicated under formula (I)

are reacted with alcohols of the formula (V) in the presence of a diluent and if appropriate in the presence of a base; or for the preparation of individual compounds of the formula (I)

d) N-halogenoacetyl-N-phenyl-ethylglycine esters of the formula

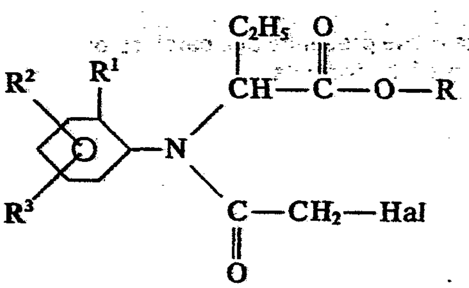

(VII)

in which

R, $R^1$, $R^2$ and $R^3$ have the meaning indicated under formula (I) and
Hal represents chlorine, bromine or iodine,

are reacted with compounds of the formula

B–X (VIII)

in which

X represents Az, cyano, thiocyano, alkylcarbonyloxy with 1 to 4 carbon atoms in the alkyl part, or the grouping $-OR^5$ or $-SR^5$, wherein Az and $R^5$ have the meaning given under formula (I) and
B represents hydrogen or an alkali metal,

in the presence of a diluent and optionally in the presence of an acid-binding agent, or

e) N-hydroxyacetyl-N-phenyl-ethylglycine esters, according to the invention, of the formula

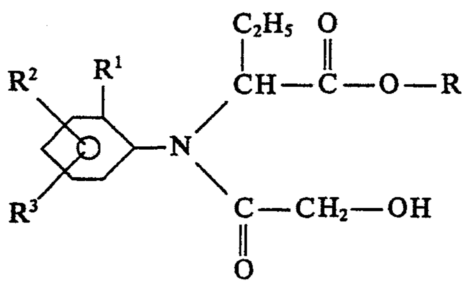

(IX)

in which

R, $R^1$, $R^2$ and $R^3$ have the meaning indicated under formula (I)

(1) are reacted, if appropriate after activation by means of an alkali metal, with halides of the formula

$$Hal-R^6 \quad (X)$$

in which

Hal has the meaning indicated above under d) and
$R^6$ represents the radical $R^5$ or the group $-SO_2R^5$ or $-COR^5$,

wherein

$R^5$ has the meaning indicated under formula (I)

in the presence of a diluent and if appropriate in the presence of an acid-binding agent, or

(2) are reacted with dihydropyrane of the formula

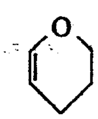

(XI)

in the presence of a diluent and if appropriate in the presence of a catalyst, or

f) N-acyloxyacetyl-N-phenyl-ethylglycine esters of the formula

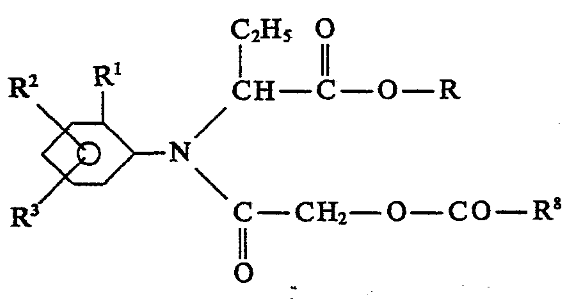

(XII)

in which

R, $R^1$, $R^2$ and $R^3$ have the meaning indicated under formula (I) and
$R^8$ represents alkyl with 1 to 4 carbon atoms,

are saponified with sodium hydroxide or potassium hydroxide in aqueous or alcoholic solution, for example in methanol or ethanol, at temperatures between 20 and 60° C and, after acidification with

compounds of the formula (V), the saponification products are esterified in the presence of an esterification catalyst, such as, for example, boron trifluoride.

3. Fungicidal agents, characterised in that they contain at least one N,N-disubstituted ethylglycine ester of the formula I.

4. Process for combating fungi, characterised in that N,N-disubstituted ethylglycine esters of the formula I are allowed to act on fungi or their environment.

5. Use of N,N-disubstituted ethylglycine esters of the formula I for combating fungi.

6. Process for the preparation of fungicidal agents, characterised in that N,N-disubstituted ethylglycine esters of the formula I are mixed with extenders and/or surface-active agents.

**Revendications**

1. Esters d'éthylglycine N,N-disubstitués de formule générale:

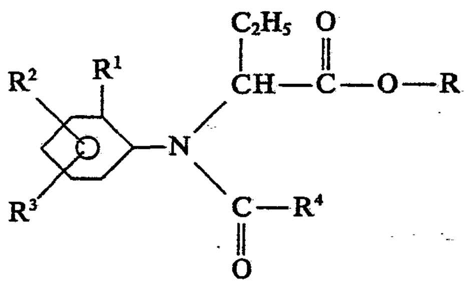

(I)

dans laquelle:

R est un reste alkyle, hydroxyalkyle, cyanalkyle, alcényle, alcynyle, halogénalkyle, cycloalkyle, cycloalkylalkyle, alkoxyalkyle, alkoxyalkoxyalkyle, acyloxyalkyle, alkylthioalkyle, alkylsulfinylalkyle, alkylsulfonylalkyle, dialkylaminoalkyle ou aralkyle éventuellement substitué,

$R^1$ est l'hydrogène, un reste alkyle ou alkoxy ou un halogène,

$R^2$ est l'hydrogène, un reste alkyle ou alkoxy ou un halogène,

$R^3$ est l'hydrogène ou un reste alkyle,

$R^4$ est un reste furyle, tétrahydrofuryle, thiényle, tétrahydrothiényle; isoxazolyle éventuellement substitué par un radical alkyle; hydroxyalkyle; alkyle, alcényle et alcynyle éventuellement substitués par un radical cyano ou thiocyano; cycloalkyle; dihalogénalkyle; ainsi que les groupements

$-CH_2-Az$ $\quad -CH_2-OR^5$ $\quad -CH_2-SR^5$ $\quad -OR^5$ $\quad -SR^5$

$-CH_2-OSO_2R^5$ $\quad -CH_2OCOR^5$ et $-CH_2-O-$ 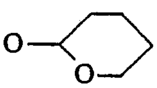

dans lesquels

$R^5$ représente des restes alkyle, alcényle, alcynyle et alkoxyalkyle éventuellement substitués, et

Az représente les restes pyrazol-1-yle, 1,2,4-triazol-1-yle et imidazol-1-yle.

2. Procédé de production d'esters d'éthylglycine N,N-disubstitués de formule (I):

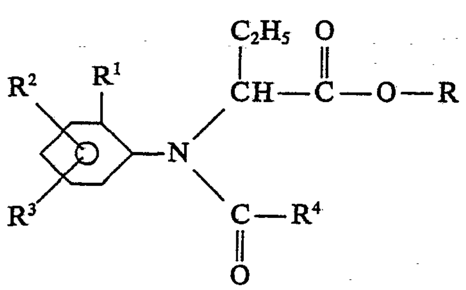

(I)

dans laquelle:

R est un reste alkyle, hydroxyalkyle, cyanalkyle, alcényle, alcynyle, halogénalkyle, cycloalkyle, cycloalkylalkyle, alkoxyalkyle, alkoxyalkoxyalkyle, acyloxyalkyle, alkylthioalkyle, alkylsulfinylalkyle, alkylsulfonylalkyle, dialkylaminoalkyle ou aralkyle éventuellement substitué,

$R^1$ est l'hydrogène, un reste alkyle ou alkoxy ou un halogène,

$R^2$ est l'hydrogène, un reste alkyle ou alkoxy ou un halogène,

$R^3$ est l'hydrogène ou un reste alkyle,

$R^4$ est un reste furyle, tétrahydrofuryle, thiényle, tétrahydrothiényle; isoxazolyle éventuellement substitué par un radical alkyle; hydroxyalkyle; alkyle, alcényle et alcynyle éventuellement substitués par un radical cyano ou thiocyano; cycloalkyle; dihalogénalkyle; ainsi que les groupements

$—CH_2—Az$ $—CH_2—OR^5$ $—CH_2—SR^5$ $—OR^5$ $—SR^5$

$—CH_2—OSO_2R^5$ $—CH_2OCOR^5$ et $—CH_2—O—$ 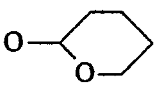

dans lesquels

$R^5$ représente des restes alkyle, alcényle, alcynyle et alkoxyalkyle éventuellement substitués, et

Az représente les restes pyrazol-1-yle, 1,2,4-triazol-1-yle et imidazol-1-yle,

caractérisé en ce que:

a) on fait réagir des esters de N-phényléthylglycine de formule:

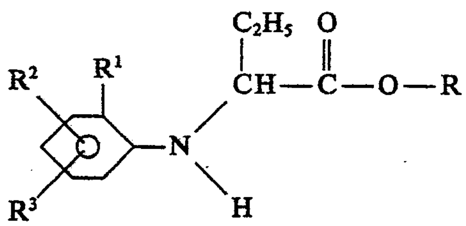

(II)

dans laquelle:

R, $R^1$, $R^2$ et $R^3$ ont la définition indiquée à propos de la formule (I),

avec des chlorures ou bromures ou des anhydrides d'acide de formules:

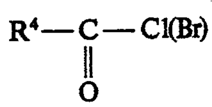

(IIIa)

ou

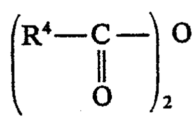

(IIIb)

dans lesquelles:

$R^4$ a la définition indiquée pour la formule (I),

en présence d'un diluant et, le cas échéant, en présence d'un accepteur d'acide, ou

b) on fait réagir des N-acyl-N-phényléthylglycines de formule:

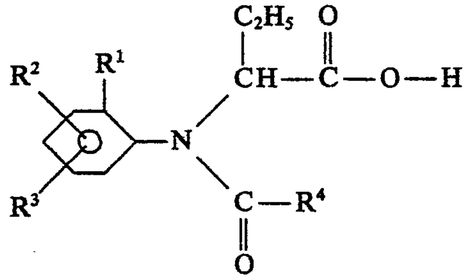

(IV)

dans laquelle:

$R^1$, $R^2$, $R^3$ et $R^4$ ont la définition indiquée pour la formule (I)

avec des alcools de formule:

$$R-O-H \qquad (V)$$

dans laquelle:

R a la définition indiquée à propos de la formule (I),

en présence d'un agent de condensation et, le cas échéant, en présence d'un diluant, ou

c) on fait réagir des chlorures de N-acyl-N-phényléthylglycines de formule:

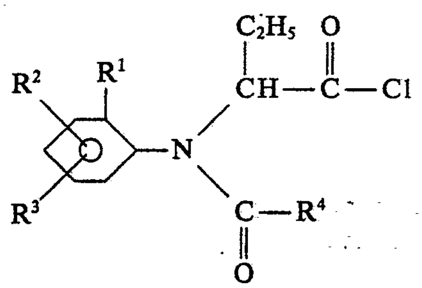

(VI)

dans laquelle:

$R^1$, $R^2$, $R^3$ et $R^4$ ont la définition indiquée pour la formule (I),

avec des alcools de formule (V) en présence d'un diluant et, le cas échéant, en présence d'une base; ou bien pour la préparation de composés particuliers de formule (I),

d) on fait réagir des esters de N-halogénacétyl-N-phényléthylglycines de formule:

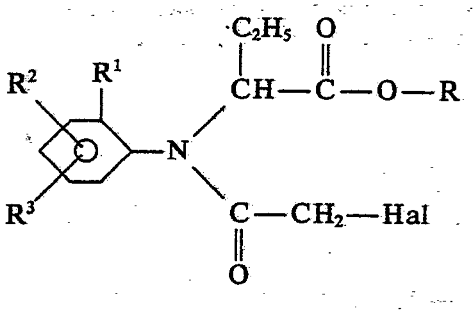

(VII)

dans laquelle:

R, $R^1$, $R^2$ et $R^3$ ont la définition indiquée pour la formule (I), et
Hal désigne le chlore, le brome ou l'iode,

avec des composés de formule:

$$B-X \qquad (VIII)$$

dans laquelle:

X représente Az, un groupe cyano, thiocyano, alkylcarbonyloxy ayant 1 à 4 atomes de carbone dans la partie alkyle et le groupement $-OR^5$ ou $-SR^5$, Az et $R^5$ ayant la définition indiquée pour la formule (I) et

B est l'hydrogène ou un métal alcalin,

en présence d'un diluant et, le cas échéant, en présence d'un accepteur d'acide, ou

e) on fait réagir des esters de N-hydroxyacétyl-N-phényléthylglycine conformes à l'invention de formule:

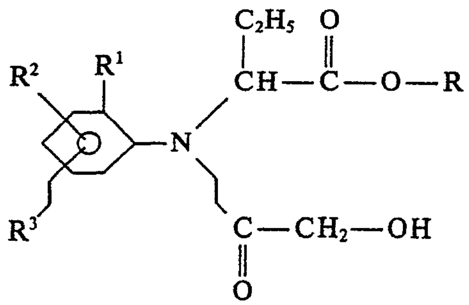

(IX)

dans laquelle:

R, $R^1$, $R^2$ et $R^3$ ont la définition indiquée pour la formule (I),

(1) éventuellement après activation au moyen d'un métal alcalin, avec des halogénures de formule:

Hal – $R^6$ (X)

dans laquelle:

Hal a la définition indiquée en d) ci-dessus, et
$R^6$ représente le reste $R^5$ ainsi que les groupes $-SO_2R^5$ et $-COR^5$,
$R^5$ ayant la définition indiquée à propos de la formule (I),

en présence d'un diluant et, le cas échéant, en présence d'un accepteur d'acide, ou bien

(2) avec le dihydropyranne de formule:

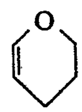

(XI)

en présence d'un diluant et, le cas échéant, en présence d'un catalyseur, ou bien

f) on saponifie un ester de N-acyloxyacétyl-N-phényléthylglycine de formule:

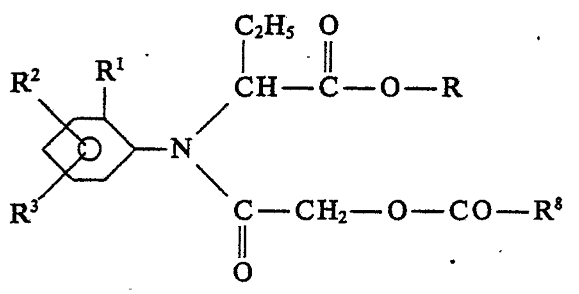

(XII)

dans laquelle:

R, $R^1$, $R^2$ et $R^3$ ont la définition indiquée pour la formule (I) et
$R^8$ est un reste alkyle ayant 1 à 4 atomes de carbone,

avec l'hydroxyde de sodium ou de potassium en solution aqueuse ou alcoolique, par exemple dans le méthanol ou l'éthanol, à des températures comprises entre 20 et 60° C et on l'estérifie après acidification avec des composés de formule (V) en présence d'un catalyseur d'estérification tel que, par exemple, le trifluorure de bore.

3. Compositions fongicides, caractérisées par une teneur en au moins un ester d'éthylglycine N,N-disubstitué de formule I.

4. Procédé de lutte contre des champignons, caractérisé en ce qu'on fait agir des esters d'éthylglycine N,N-disubstitués de formule I sur des champignons ou sur leur milieu.

5. Utilisation d'esters d'éthylglycine N,N-disubstitués de formule I pour la lutte contre des champignons.

6. Procédé de préparation de compositions fongicides, caractérisé en ce qu'on mélange des esters d'éthylglycine N,N-disubstitués de formule I avec des diluants et/ou des agents tensio-actifs.